# EUROPEAN PATENT APPLICATION

(11) **EP 4 763 046 A1**
(43) Date of publication of application: **24.06.2026**
(21) Application number: 25224912.3
(22) Date of filing: 18.12.2025
(51) Int. Cl.: A61B 1/05, A61B 1/06

(54) **IMAGING DEVICE, IMAGING SYSTEM, AND METHOD FOR MANUFACTURING THE IMAGING SYSTEM**

(30) Priority: 20.12.2024 CN 202411898664; 14.07.2025 CN 202510969172; 24.11.2025 CN 202511740159; 24.11.2025 CN 202511734874
(71) Applicant: Taizhou Guanyu Technology Co., Ltd., Taizhou City, Zhejiang Province (CN)
(72) Inventor: HSU, KUO-CHENG, 406 TAICHUNG CITY (TW); CHEN, HUEI-SIOU, 103 TAIPEI CITY (TW); WEI, LI-CHEN, 406 TAICHUNG CITY (TW); HSU, SHUN-CHENG, TAIZHOU CITY (CN)
(74) Representative: Michalski Hüttermann & Partner mbB

(57) **Abstract**

The disclosure relates to an imaging device, an imaging system, and a method for manufacturing the imaging system. The imaging system includes a processor and a distal system. The distal system is electrically connected to the processor. The distal system includes an image sensor structure and a light emitting device. The light emitting device includes an organic light emitting device (OLED), a micro light emitting diode (micro-LED), a quantum dot light emitting diode (QLED), or any combination thereof. The organic light emitting device includes an electrode, a semi-reflective surface, a fully reflective surface, and an organic light emitting layer. The organic light emitting layer is disposed above the electrode between the semi-reflective surface and the fully reflective surface, and a distance between the semi-reflective surface and the fully reflective surface defines a micro resonant cavity.

## Description

### TECHNICAL FIELD

The disclosure relates to an imaging device, an imaging system, and a method for manufacturing the imaging system. More particularly, the disclosure relates to an imaging device including an organic light emitting device, an imaging system including an organic light emitting device, and a method for manufacturing the imaging system.

### BACKGROUND

In recent years, the light sources of flexible endoscopes mainly rely on external light sources transmitted via optical fibers or micro light emitting diode arrays at the probe ends. However, the current endoscope structures are still limited by low resolution, bulky structure, high power consumption, and complex optical paths. These shortcomings limit the development of ultrathin, low heat, rapidly switchable multispectral and structured light endoscopic systems, and thus there is an urgent need to develop endoscopes that provide low power, low heat, surface emitting, narrow band, and high frequency structured light sources within an extremely small volume.

### SUMMARY

In this disclosure, an imaging device includes a holding portion and a distal end, the distal end is connected to the holding portion, and the distal end includes an image sensor and an organic light emitting device (OLED). The organic light emitting device includes a substrate, an electrode, a first reflective surface, a second reflective surface, and an organic light emitting layer. The electrode is disposed on the substrate. The organic light emitting layer is disposed between the first reflective surface and the second reflective surface. For light emitted from the organic light emitting layer, the first reflective surface has a reflectance equal to or greater than 30%, and the second reflective surface has a reflectance greater than the reflectance of the first reflective surface, and a distance between the first reflective surface and the second reflective surface defines a micro resonant cavity.

In this disclosure, an imaging system includes a processor and a distal system. The distal system is electrically connected to the processor. The distal system includes an image sensor structure and a light emitting device. The light emitting device includes an organic light emitting device (OLED), a micro light emitting diode (micro-LED), a quantum dot light emitting diode (QLED), or any combination thereof. The organic light emitting device includes an electrode, a semi-reflective surface, a fully reflective surface, and an organic light emitting layer. The organic light emitting layer is disposed above the electrode between the semi-reflective surface and the fully reflective surface, and a distance between the semi-reflective surface and the fully reflective surface defines a micro resonant cavity.

In this disclosure, a method for manufacturing an imaging system includes: manufacturing a distal system; and electrically connecting the distal system to a processor. Manufacturing the distal system includes: providing a housing having a first hollow conduit; manufacturing a light emitting device, the light emitting device comprising an OLED, a micro-LED, a QLED, or any combination thereof; and disposing the light emitting device within the first hollow conduit.

In some embodiments, the organic light emitting device is configured to switchably emit colored light, narrow band imaging (NBI) light, and structured light toward outside of the distal end.

In some embodiments, the imaging device further includes a wire structure and a bending portion, and the bending portion is connected to the distal end, wherein the wire structure passes through a hollow conduit of the bending portion and a hollow conduit of the holding portion to transmit a control signal to the organic light emitting device to switch between emitting the colored light, the NBI light and the structured light.

In some embodiments, the imaging device further includes a wire structure and a bending portion, and the bending portion is connected to the distal end, wherein the wire structure passes through a hollow conduit of the bending portion and a hollow conduit of the holding portion to transmit power to the organic light emitting device and the image sensor, or to transmit image information captured by the image sensor to a device outside the holding portion.

In some embodiments, the distal end does not include a light emitting diode (LED) or a color filter.

In some embodiments, the light emitting device further includes a first microlens structure disposed on a light emitting surface of the OLED and a second microlens structure disposed on a light emitting surface of the micro-LED.

In some embodiments, the light emitting device is configured to switchably emit colored light having two or more colors, NBI light having a full width at half maximum (FWHM) less than 30 nm, and structured light having a predetermined pattern.

In some embodiments, the light emitting device further includes a substrate, an organic light emitting device array (OLED array) disposed on the substrate, and a micro light emitting diode array (micro-LED array) disposed on the substrate, the OLED array includes the OLED, and the micro-LED array includes the micro-LED.

In some embodiments, the micro-LED array includes a plurality of micro-LEDs, and light emitted from the micro-LED array having one or more colors is used to compensate for luminous efficiency of light emitted from a plurality of OLEDs of the OLED array having one or more colors.

In some embodiments, the light emitting device further includes a transparent encapsulation layer covering the OLED array and exposing the micro-LED array.

In some embodiments, the image sensor structure includes a plurality of first image sensors integrated into the OLED array and a plurality of second image sensors integrated into the micro-LED array, and the first image sensors and the second image sensors are all disposed on the substrate.

In some embodiments, the distal system further includes a first hollow conduit and a second hollow conduit, the light emitting device is disposed in the first hollow conduit, and the image sensor structure is disposed in the second hollow conduit.

In some embodiments, the distal system further includes a transparent protection plate covering an opening of the first hollow conduit and a lens disposed between the transparent protection plate and the light emitting device.

In some embodiments, the distal system is configured to capture image information of an object under test and transmit the image information to the processor, and the processor is configured to process the image information to generate an image of an appearance of the object or a 3D structural simulation result of the object.

In some embodiments, the imaging system further includes an augmented reality glasses (AR glasses), wherein the AR glasses includes the processor.

In some embodiments, the imaging system further includes an AR glasses, wherein the AR glasses is configured to receive the image of the appearance of the object and/or the 3D structural simulation result of the object, and after processed by the processor using a swarm algorithm, to present a real-time image in a display area of the AR glasses.

In some embodiments, the distal system includes a colored light source, an NBI light source, and a structured light source, and the image sensor is configured to capture image information.

In some embodiments, the processor transmit a control signal to control the organic light emitting device to switch between emitting colored light, NBI light, and structured light, so as to implement the colored light source, the NBI light source, and the structured light source of the distal system.

In some embodiments, the distal end does not include a light emitting diode (LED) or a color filter.

In some embodiments, the method further includes: disposing a first microlens structure on a light emitting surface of the OLED; and disposing a second microlens structure on a light emitting surface of the micro-LED.

In some embodiments, the method further includes: disposing a lens between an opening of the first hollow conduit and the light emitting device; and covering the opening of the first hollow conduit with a transparent protection plate.

In some embodiments, the method further includes disposing an image sensor structure within a second hollow conduit of the housing of the distal system.

In some embodiments, manufacturing the light emitting device includes: providing a substrate; disposing an OLED array comprising the OLED and a micro-LED array comprising the micro-LED on the substrate; and covering a transparent encapsulation layer on the OLED array and exposing the micro-LED array.

In some embodiments, the method further includes: disposing an image sensor structure on the substrate, including: integrating a plurality of first image sensors into the OLED array; and integrating a plurality of second image sensors into the micro-LED array.

### BRIEF DESCRIPTION OF THE DRAWINGS

FIG. 1 is a top view exemplarily showing an intermediate product of an OLED.
FIG. 2A is a cross sectional view exemplarily showing an OLED.
FIG. 2B is a cross sectional view exemplarily showing an OLED.
FIG. 2C is a top view exemplarily showing a portion of an OLED.
FIG. 3 is a diagram exemplarily showing an imaging device.
FIG. 4A is a diagram exemplarily showing an imaging system.
FIG. 4B is a diagram exemplarily showing an imaging system.
FIG. 4C is a diagram exemplarily showing an imaging system.
FIG. 5A is a diagram exemplarily showing a distal system.
FIG. 5B is a diagram exemplarily showing a distal system.
FIG. 5C is a top view exemplarily showing a light emitting device.
FIG. 5D is a top view exemplarily showing an image sensor structure.
FIG. 6A is a cross sectional view exemplarily showing a light emitting device.
FIG. 6B is a cross sectional view exemplarily showing a light emitting device.
FIG. 6C is a cross sectional view exemplarily showing a micro-LED.
FIG. 6D is a cross sectional view exemplarily showing a micro-LED.
FIG. 6E is a cross sectional view exemplarily showing a micro-LED.
FIG. 7A is a top view exemplarily showing a portion of a micro-LED array.
FIG. 7B is a top view exemplarily showing a portion of a micro-LED array.
FIG. 7C is a top view exemplarily showing a portion of an OLED array.
FIG. 7D is a top view exemplarily showing a portion of an OLED array.
FIG. 7E is a top view exemplarily showing a portion of an OLED array.
FIG. 7F is a top view exemplarily showing a portion of an OLED array.
FIG. 8A is a diagram exemplarily showing a distal system.
FIG. 8B is a cross sectional view exemplarily showing a distal system.
FIG. 8C is a top view exemplarily showing a light emitting device.
FIG. 9A is a top view exemplarily showing a portion of a micro-LED array.
FIG. 9B is a top view exemplarily showing a portion of a micro-LED array.
FIG. 9C is a top view exemplarily showing a portion of an OLED array.
FIG. 9D is a top view exemplarily showing a portion of an OLED array.
FIG. 10A is a diagram exemplarily showing a light path of a distal system.
FIG. 10B is a diagram exemplarily showing a light path of a distal system.
FIG. 11, (a) to (g), are diagrams exemplarily showing patterns of structured light.
FIGS. 12A-12D are diagrams exemplarily showing a method for manufacturing an imaging system.

### DETAILED DESCRIPTION

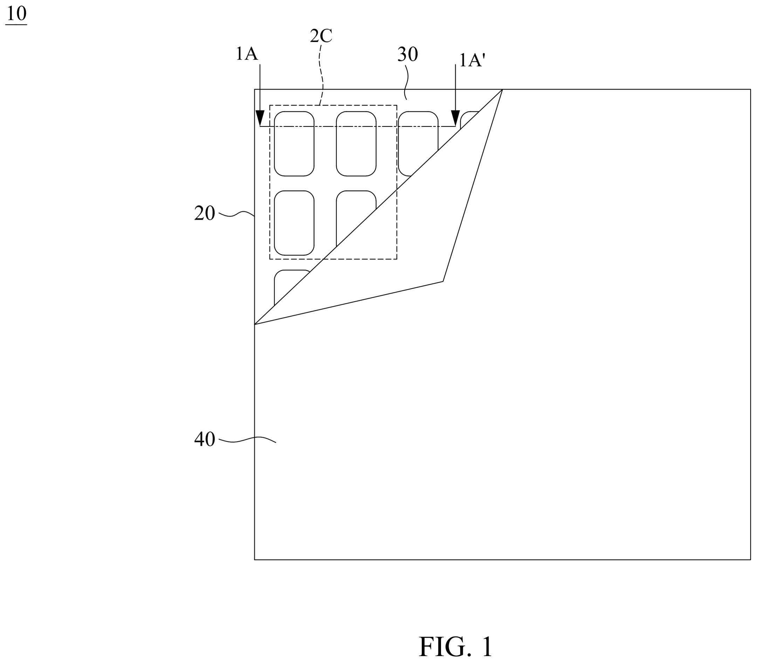

FIG. 1 is a top view exemplarily showing an intermediate product of an OLED 10.The OLED 10 has a light emitting layer 20 and a covering layer 40 disposed on the light emitting layer 20.For the light emitting layer 20, a spacer structure 30 may be designed to provide a recess array for accommodating a light emitting pixel array. In some embodiments, the spacer structure 30 may include a protrusion 310.In some embodiments, the spacer structure 30 may include a light sensitive material.

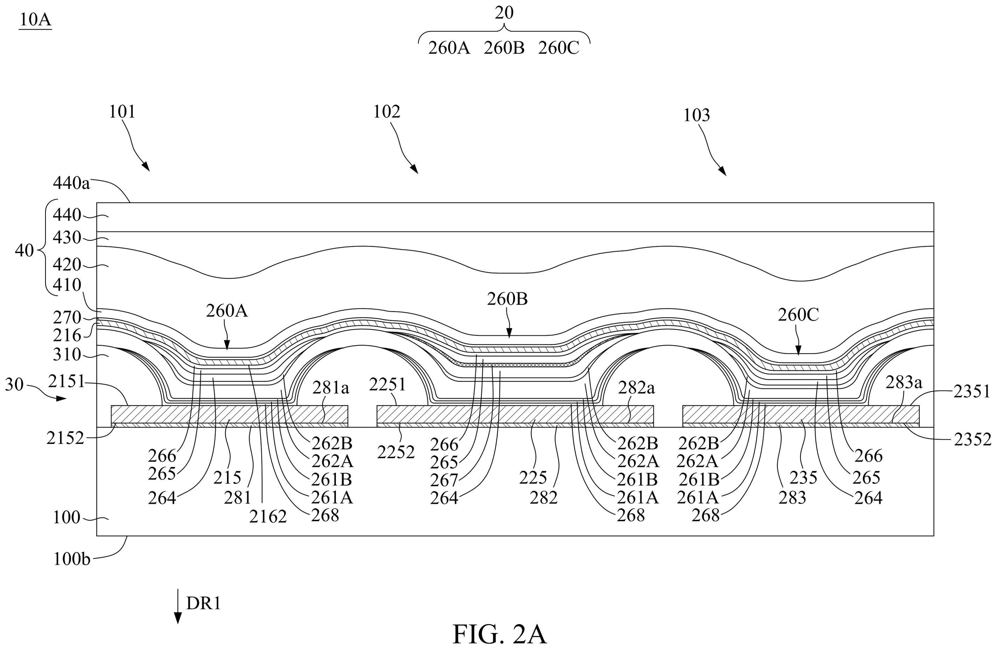

FIG. 2A is a cross sectional view exemplarily showing an OLED 10A. In some embodiments, FIG. 2A is a cross sectional view exemplarily taken along line 1A-1A' in FIG. 1. In some embodiments, FIG. 2A is a cross sectional view exemplarily taken along line 1A-1A' in FIG. 1 and illustrating only a light emitting region. The spacer structure 30 has several protrusions 310 to define a light emitting pixel pattern. A recess is located between two adjacent protrusions 310 and provides space for accommodating a light emitting pixel. One skilled in the art should understand that, when observed from the cross sectional view of FIG. 2A, the protrusions 310 are illustrated as disconnected, but as observed in the top view of FIG. 1, they are capable of being connected to each other through other portions of the spacer structure 30.

As shown in FIG. 2A, in some embodiments, the OLED 10A is, for example, a light emitting device including an organic light emitting diode structure. In some embodiments, the OLED 10 includes a plurality of organic light emitting units (also referred to as light emitting pixels), for example, includes at least an organic light emitting unit 101 (also referred to as a first organic light emitting unit), an organic light emitting unit 102 (also referred to as a second organic light emitting unit), and an organic light emitting unit 103 (also referred to as a third organic light emitting unit). In some embodiments, the organic light emitting units 101, 102, and 103 are located between the protrusions 310 and above a substrate 100.The organic light emitting units 101, 102, and 103 may emit light of the same wavelength or light of different wavelengths.

In some embodiments, the OLED 10 includes a substrate 100, an electrode 215, an electrode 225, an electrode 235, an electrode 216, the light emitting layer 20, a reflective layer 281, a reflective layer 282, a reflective layer 283, the spacer structure 30, and the covering layer 40.

In some embodiments, the substrate 100 may include a transistor array arranged corresponding to the light emitting pixels in the light emitting layer 20.The substrate 100 may include several capacitors. In some embodiments, more than one transistor is arranged together with a capacitor and a light emitting pixel to form a circuit. In some embodiments, the substrate 100 may include glass.

In some embodiments, the electrode 215, the electrode 225, and the electrode 235 are disposed on the substrate 100.In some embodiments, the electrodes 215, 225, and 235 are anodes. In some embodiments, the electrodes 215, 225, and 235 include a metal material, such as Ag, Al, Mg, Au, AlCu alloy, AgMo alloy, or the like. In some embodiments, the electrodes 215, 225, and 235 include indium tin oxide (ITO), indium zinc oxide (IZO), or any other suitable material.

In some embodiments, the light emitting layer 20 includes an organic light emitting layer 260A (also referred to as a first organic light emitting layer), an organic light emitting layer 260B (also referred to as a second organic light emitting layer), and an organic light emitting layer 260C (also referred to as a third organic light emitting layer). In some embodiments, the organic light emitting layer 260A is disposed on the electrode 215, the organic light emitting layer 260B is disposed on the electrode 225, and the organic light emitting layer 260C is disposed on the electrode 235. In some embodiments, a thickness of the organic light emitting layer 260A, a thickness of the organic light emitting layer 260B, and a thickness of the organic light emitting layer 260C are all different. In some embodiments, the thickness of the organic light emitting layer 260B is greater than the thickness of the organic light emitting layer 260A, and the thickness of the organic light emitting layer 260A is greater than the thickness of the organic light emitting layer 260C.

In some embodiments, the organic light emitting layers 260A, 260B, and 260C emit light of the same color or light of different colors. In some embodiments, a light emitting wavelength of the organic light emitting layer 260B is greater than a light emitting wavelength of the organic light emitting layer 260A, and the light emitting wavelength of the organic light emitting layer 260A is greater than a light emitting wavelength of the organic light emitting layer 260C. In some embodiments, the organic light emitting layer 260A emits green light, the organic light emitting layer 260B emits red light, and the organic light emitting layer 260C emits blue light.

In some embodiments, organic material layers of the organic light emitting layers 260A, 260B, and 260C include an organic material, which may be disposed in any of the organic material layers of the organic light emitting layers 260A, 260B, and 260C based on different implementations. In some embodiments, the organic material has an absorptivity of 50% or higher, 60% or higher, 70% or higher, 80% or higher, 90% or higher, or 95% or higher for a specific wavelength. In some embodiments, the specific wavelength is not greater than 400 nm, 350 nm, 300 nm, 250 nm, 200 nm, 150 nm, or 100 nm.

As shown in FIG. 2A, in some embodiments, the organic light emitting unit 101 includes the electrode 215, the organic light emitting layer 260A, and the electrode 216. In some embodiments, the organic light emitting layer 260A includes a plurality of organic material layers, such as a hole injection layer (HIL) 261A, a hole injection layer (HIL) 261B, a hole transport layer (HTL) 262A, a hole transport layer (HTL) 262B, an organic emissive layer (EM) 264, an electron transport layer (ETL) 265, and an electron injection layer (EIL) 266. In some embodiments, the electrode 216 is disposed above the organic light emitting layer 260A.

As shown in FIG. 2A, in some embodiments, the organic light emitting unit 102 includes the electrode 225, the organic light emitting layer 260B, and the electrode 216. In some embodiments, the organic light emitting layer 260B includes a plurality of organic material layers, such as a hole injection layer (HIL) 261A, a hole injection layer (HIL) 261B, a hole transport layer (HTL) 262A, a hole transport layer (HTL) 262B, an organic emissive layer (EM) 264, an electron transport layer (ETL) 265, and an electron injection layer (EIL) 266. In some embodiments, the electrode 216 is disposed above the organic light emitting layer 260B.

As shown in FIG. 2A, in some embodiments, the organic light emitting unit 103 includes the electrode 235, the organic light emitting layer 260C, and the electrode 216. In some embodiments, the organic light emitting layer 260C includes a plurality of organic material layers, such as a hole injection layer (HIL) 261A, a hole injection layer (HIL) 261B, a hole transport layer (HTL) 262A, a hole transport layer (HTL) 262B, an organic emissive layer (EM) 264, an electron transport layer (ETL) 265, and an electron injection layer (EIL) 266. In some embodiments, the electrode 216 is disposed above the organic light emitting layer 260C.

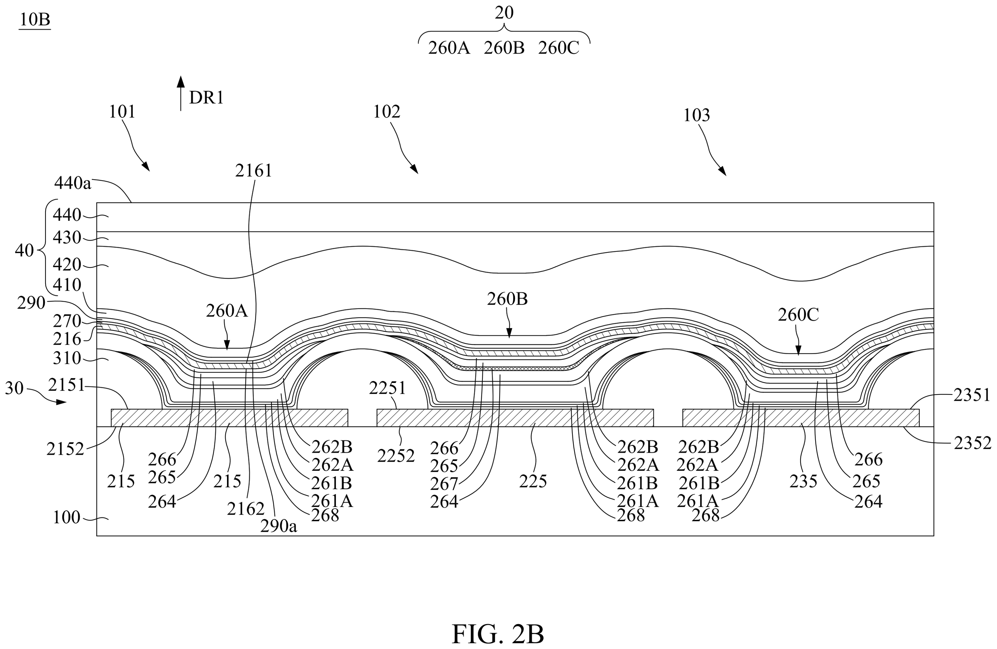

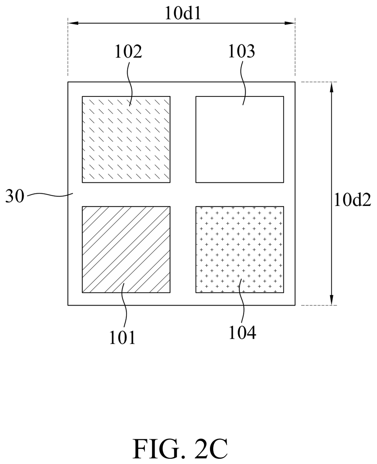

In some embodiments, the electrode 216 contacts the organic light emitting layers 260A, 260B, and 260C. The electrode 216 may be a continuous film as shown in FIG. 2 and is disposed above the organic light emitting layers 260A, 260B, and 260C and the protrusions 310.In some embodiments, the electrodes 216 may be further disposed on the spacer structure 30.In some embodiments, the electrode 216 is a common electrode for all light emitting pixels in the light emitting layer 20.In some embodiments, the electrode 216 includes a metal material, such as Ag, Al, Mg, Au, AlCu alloy, AgMo alloy, or the like. In some embodiments, the electrode 216 includes ITO, IZO, or any other suitable material. In other words, the electrode 216 is a common electrode for several organic light emitting units. In some embodiments, the electrode 216 is a common electrode for all organic light emitting units in the OLED 10A.

In some embodiments, the reflective layer 281 is disposed between the substrate 100 and the electrode 215. In some embodiments, a surface 2151 of the electrode 215 faces the electrode 216, and a surface 2152 of the electrode 215 opposite to the surface 2151 faces the substrate 100 and contacts the reflective layer 281. In some embodiments, the reflective layer 281 includes a reflective surface 281a (also referred to as a first reflective surface), the electrode 216 includes a surface 2162 (also referred to as a second reflective surface), the reflective surface 281a faces the organic light emitting layer 260A, and the surface 2162 faces the organic light emitting layer 260A. In some embodiments, the electrode 215 is a transparent electrode, and the reflective surface 281a is used to further reflect the light emitted from the organic light emitting layer 260A. In some embodiments, the reflective surface 281a faces away from a light emitting surface (e. g. , a surface 100b) of the OLED 10A, and the reflective surface 281a is closer to the light emitting surface of the OLED 10A than the surface 2162.In some embodiments, the OLED 10A emits light along a light emitting direction DR1. In some embodiments, for the light emitted from the organic light emitting layer 260A, the reflective surface 281a has a reflectance of 30% or higher, such as 40% or higher, 50% or higher, 60% or higher, or 70% or higher. In some embodiments, for the light emitted from the organic light emitting layer 260A, the surface 2162 (or the second reflective surface) has a reflectance greater than the reflectance of the reflective surface 281a (or the first reflective surface), such as 80% or higher, 85% or higher, 90% or higher, or 95% or higher. In some embodiments, a distance between the reflective surface 281a (or the first reflective surface) and the surface 2162 (or the second reflective surface) defines a micro resonant cavity.

In some embodiments, the reflective layer 282 is disposed between the substrate 100 and the electrode 225. In some embodiments, a surface 2251 of the electrode 225 faces the electrode 216, and a surface 2252 of the electrode 225 opposite to the surface 2251 faces the substrate 100 and contacts the reflective layer 282.In some embodiments, the reflective layer 282 includes a reflective surface 282a (also referred to as a third reflective surface), and the reflective surface 282a faces the organic light emitting layer 260B. In some embodiments, the electrode 225 is a transparent electrode, and the reflective surface 282a is used to further reflect the light emitted from the organic light emitting layer 260B. In some embodiments, the reflective surface 282a faces away from the light emitting surface of the OLED 10A, and the reflective surface 282a is closer to the light emitting surface of the OLED 10A than the surface 2162.In some embodiments, for the light emitted from the organic light emitting layer 260B, the reflective surface 282a has a reflectance of 30% or higher, such as 40% or higher, 50% or higher, 60% or higher, or 70% or higher. In some embodiments, for the light emitted from the organic light emitting layer 260B, the surface 2162 (or the second reflective surface) has a reflectance greater than the reflectance of the reflective surface 282a (or the first reflective surface), such as 80% or higher, 85% or higher, 90% or higher, or 95% or higher.

In some embodiments, the reflective layer 283 is disposed between the substrate 100 and the electrode 235. In some embodiments, a surface 2351 of the electrode 235 faces the electrode 216, and a surface 2352 of the electrode 235 opposite to the surface 2351 faces the substrate 100 and contacts the reflective layer 283. In some embodiments, the reflective layer 283 includes a reflective surface 283a (also referred to as a third reflective surface), and the reflective surface 283a faces the organic light emitting layer 260C. In some embodiments, the electrode 235 is a transparent electrode, and the reflective surface 283a is used to further reflect the light emitted from the organic light emitting layer 260C. In some embodiments, the reflective surface 283a faces away from the light emitting surface of the OLED 10A, and the reflective surface 283a is closer to the light emitting surface of the OLED 10A than the surface 2162.In some embodiments, for the light emitted from the organic light emitting layer 260C, the reflective surface 283a has a reflectance of 30% or higher, such as 40% or higher, 50% or higher, 60% or higher, or 70% or higher. In some embodiments, for the light emitted from the organic light emitting layer 260C, the surface 2162 (or the second reflective surface) has a reflectance greater than the reflectance of the reflective surface 283a (or the first reflective surface), such as 80% or higher, 85% or higher, 90% or higher, or 95% or higher.

In some embodiments, when a reflective surface (or a first reflective surface) has a reflectance of 30% or higher, the FWHM of an emission spectrum peak of the organic light emitting layer can be reduced by 10% or more. In some embodiments, when a reflective surface (or a first reflective surface) has a reflectance of 40% or higher, the FWHM of an emission spectrum peak of the organic light emitting layer can be reduced by 15% or more. In some embodiments, when a reflective surface (or a first reflective surface) has a reflectance of 50% or higher, the FWHM of an emission spectrum peak of the organic light emitting layer can be reduced by 20% or more. In some embodiments, when a reflective surface (or a first reflective surface) has a reflectance of 60% or higher, the FWHM of an emission spectrum peak of the organic light emitting layer can be reduced by 25% or more.

In some embodiments, when a reflective surface (or a first reflective surface) has a reflectance of 30% or higher, an emission divergence angle of the organic light emitting layer is about ±60 degrees or less. In some embodiments, when a reflective surface (or a first reflective surface) has a reflectance of 40% or higher, an emission divergence angle of the organic light emitting layer is about ±50 degrees or less. In some embodiments, when a reflective surface (or a first reflective surface) has a reflectance of 50% or higher, an emission divergence angle of the organic light emitting layer is about ±40 degrees or less. In some embodiments, when a reflective surface (or a first reflective surface) has a reflectance of 60% or higher, an emission divergence angle of the organic light emitting layer is about ±30 degrees or less.

In some embodiments, each of the reflective layers 281, 282, and 283 includes a reflective metal or a non-conductive reflective material. In some embodiments, each of the reflective layers 281, 282, and 283 includes Ag, a distributed Bragg reflector (DBR), or any other suitable reflective material. In some embodiments, the greater the thickness of a reflective metal, the higher the reflectance of the reflective metal. In some embodiments, the more layers in a DBR, the higher the reflectance of the DBR.

In some embodiments, the spacer structure 30 is disposed on the substrate 100 and partially covers the electrodes 215, 225, and 235. In some embodiments, the spacer structure 30 is disposed between the organic light emitting layers 260A, 260B, and 260C. In some embodiments, the spacer structure 30 may include the protrusions 310.In some embodiments, a pattern of the spacer structure 30 is designed based on a pixel arrangement. In some embodiments, the spacer structure 30 is used as a pixel defined layer (PDL). In some embodiments, the protrusions 310 define pixel regions. In some embodiments, each protrusion 310 fills into a gap between adjacent two of the electrodes 215, 225, and 235. Each of the electrodes 215, 225, and 235 is partially covered by the protrusions 310.In some embodiments, the spacer structure 30 includes an organic insulating material. In some embodiments, the spacer structure 30 includes a light sensitive material. In some embodiments, the spacer structure 30 may further include quantum dots having excellent light absorption efficiency. In some embodiments, the spacer structure 30 may further include a carbon black material, such as carbon black nanoparticles, conductive fibers containing carbon black, or the like. In some embodiments, the spacer structure 30 may further include a black body material having an absorptivity of 90%, 95%, 99%, 99.5%, or 99.9% or higher for visible light.

In some embodiments, the organic material has an absorptivity of 50% or higher, 60% or higher, 70% or higher, 80% or higher, 90% or higher, or 95% or higher for a specific wavelength. In some embodiments, the specific wavelength is not greater than 400 nm, 350 nm, 300 nm, 250 nm, 200 nm, 150 nm, or 100 nm.

In some embodiments, the covering layer 40 includes a capping layer 410, an encapsulation layer 420, a filling layer 430, and a cover plate 440.In some embodiments, the capping layer 410 is disposed on the electrode 216, and is substantially conformal to a non-planar upper surface of the electrode 216. The capping layer 410 may include a dielectric material or an inorganic insulating material, such as silicon oxide. In some embodiments, the capping layer 410 may include a hole transport layer material for extracting light lost inside the OLED to increase the light emitting efficiency. The capping layer 410 may also be referred to as a light extraction layer.

In some embodiments, the encapsulation layer 420 is disposed on the capping layer 410, and is substantially conformal to a non-planar upper surface of the capping layer 410.The encapsulation layer 420 may include an oxide, such as silicon oxide. In some embodiments, the encapsulation layer 420 is substantially conformal to the non-planar upper surface of the capping layer 410, and includes a plurality of recesses corresponding to the organic light emitting layers 260A, 260B, and 260C. The encapsulation layer 420 may include an organic polymer material, such as an epoxy-based material.

In some embodiments, the filling layer 430 is disposed on the encapsulation layer 420, and a lower surface of the filling layer 430 is substantially conformal to a non-planar upper surface of the encapsulation layer 420.The filling layer 430 may also be referred to as a planarization layer. The filling layer 430 may include an organic polymer material, such as an epoxy-based material.

In some embodiments, the cover plate 440 is disposed on a planar upper surface of the filling layer 430.The cover plate 440 may also be referred to as a protection layer. The cover plate 440 may include a transparent hard cover plate, such as a glass plate. The cover plate 440 may be used to prevent components of the OLED from being exposed to external moisture, which may lead to component failures and inability to emit light.

In some embodiments, the inorganic barrier layer 268 is disposed between the electrodes 215, 225, and 235 and the organic light emitting layers 260A, 260B, and 260C. In some embodiments, a side surface of the inorganic barrier layer 268 contacts a protrusion 310.In some embodiments, the inorganic barrier layer 268 substantially completely covers interfaces between the electrodes 215, 225, and 235 and the organic light emitting layers 260A, 260B, and 260C. In some embodiments, the inorganic barrier layer 268 includes a transition metal oxide. In some embodiments, the inorganic barrier layer 268 includes MoO₃. In some embodiments, the inorganic barrier layer 268 has a thickness of 100 Å or less. In some embodiments, a ratio of the thickness of the inorganic barrier layer 268 to a thickness of the electrodes 215, 225, and 235 is less than 0.1, 0.06, or 0.03. In some embodiments, the inorganic barrier layer 268 together with the hole injection layers 261A and 261B may constitute hole injection layers of the organic light emitting layers 260A, 260B, and 260C.

In some embodiments, the inorganic barrier layer 270 contacts the capping layer 410.In some embodiments, the inorganic barrier layer 270 covers the electrode 216. In some embodiments, the capping layer 410 is disposed on the inorganic barrier layer 270, and is separated from the electrode 216 by the inorganic barrier layer 270.In some embodiments, the inorganic barrier layer 270 substantially completely covers an interface between the electrode 216 and the capping layer 410.In some embodiments, the inorganic barrier layer 270 includes a transition metal oxide. In some embodiments, the inorganic barrier layer 270 includes MoO₃. In some embodiments, the inorganic barrier layer 270 has a thickness of 100 Å or less. In some embodiments, a ratio of the thickness of the inorganic barrier layer 270 to a thickness of the electrode 216 is less than 0.15, 0.1, or 0.05. In some embodiments, a ratio of the thickness of the inorganic barrier layer 270 to a thickness of the capping layer 410 is less than 0.5, 0.3, or 0.15.

FIG. 2B is a cross sectional view exemplarily showing an OLED 10B. In some embodiments, FIG. 2B is a cross sectional view exemplarily showing the OLED 10 of FIG. 1. In some embodiments, FIG. 2B is a cross sectional view exemplarily taken along line 1A-1A' in FIG. 1. In some embodiments, FIG. 2B is a cross sectional view exemplarily taken along line 1A-1A' in FIG. 1 and illustrating only a light emitting region. FIG. 2B has a structure similar to the structure of FIG. 2A, with a difference described as follows.

In some embodiments, the OLED 10B includes a reflective layer 290, the electrode 216 is a transparent electrode, and the reflective layer 290 is disposed between the capping layer 410 and the electrode 216. In some embodiments, the reflective layer 290 includes a non-conductive material, such as a DBR. In some embodiments, the reflective layer 290 includes a plurality of pairs of non-conductive material layers, and a refractive index difference between each pair of non-conductive material layers is 0.4 or more. In some embodiments, the reflective layer 290 includes a reflective surface 290a. In some embodiments, a surface 2161 of the electrode 216 faces the capping layer 410 and contacts the reflective layer 290.In some embodiments, the surface 2162 of the electrode 216 faces the electrodes 215, 225, and 235. In some embodiments, for the light emitted from the organic light emitting layers 260A, 260B, and 260C, the reflective surface 290a has a reflectance of 30% or higher, such as 40% or higher, 50% or higher, 60% or higher, or 70% or higher. In some embodiments, a distance between the reflective surface 290a and the surfaces 2151, 2251, and 2351 of the electrodes 215, 225, and 235 defines a micro resonant cavity. In some embodiments, a light emitting surface of the OLED 10B is a surface 440a of the cover plate 44. In some embodiments, the OLED 10B emits light along a light emitting direction DR1.

FIG. 2C is a top view exemplarily showing a portion of an OLED. In some embodiments, FIG. 2C is a top view exemplarily showing a portion 2C of the OLED 10 of FIG. 1. In some embodiments, FIG. 2C is a diagram exemplarily showing the light emitting units 101, 102, 103, and 104 of the OLED 10, 10A, or 10B.

In some embodiments, the OLED shown in FIG. 2C may include the light emitting units 101, 102, 103, and 104 having the same or different light emitting wavelengths. In some embodiments, the light emitting units 101, 102, 103, and 104 are sub-pixels, and a plurality of light emitting units 101, 102, 103, and 104 (or sub-pixels) constitute a pixel. In some embodiments, the entire pixel has a length 10d1 and a width 10d2 of about 3 µm to 5 µm, such as 4 µm.

In some embodiments, each of the light emitting units 101, 102, 103, and 104 may emit light with a wavelength of about 400 nm to 1100 nm. In some embodiments, the pixel constituted by the light emitting units 101, 102, 103, and 104 may emit light with a wavelength of about 400 nm to 1100 nm. In some embodiments, the light emitting units 101, 102, and 103 emit green light, red light, and blue light, respectively, and the light emitting unit 104 emits light with a wavelength of about 415 nm and/or 530 nm (suitable for an NBI light source).

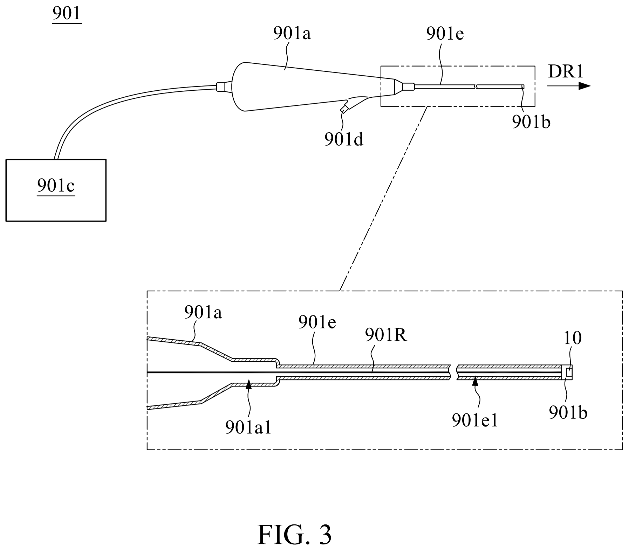

FIG. 3 is a diagram exemplarily showing an imaging device 901. In some embodiments, FIG. 3 shows a cross sectional view of the imaging device 901. In some embodiments, the imaging device 901 may include an endoscope, such as a single-use endoscope.

In some embodiments, the imaging device 901 includes a holding portion 901a, a distal end 901b, a device 901c, a working channel inlet 901d, a bending portion 901e, and a wire structure 901R.

In some embodiments, the holding portion 901a is, for example, a grip. In some embodiments, the holding portion 901a includes a hollow conduit 901a1. In some embodiments, the holding portion 901a is configured to be held by the left hand or the right hand, providing a stable base for advancement and operation. In some embodiments, the holding portion 901a may include a control lever (not shown) for controlling the bending angle of a lens end (i.e., the distal end 901b and the bending section 901e) by moving the control lever up and down. In some embodiments, the working channel inlet 901d is located at the bottom of the holding portion 901a and communicates with the working channel inside the hollow conduit 901a1 of the holding portion 901a for delivering/aspirating fluids or inserting other endoscopic instruments.

In some embodiments, the distal end 901b includes an image sensor (not shown), a light source, and a working channel outlet (not shown). In some embodiments, the distal end 901b is, for example, a probe of the endoscope configured to insert into a living organism for detection. The image sensor may include a camera, and the light source of the imaging device 901 may include an OLED 10.In some embodiments, the light source of the imaging device 901 may include the OLED 10A or 10B as described above. In some embodiments, the OLED 10 may be or include the OLED 10A or 10B as described above. In some embodiments, the OLED 10 (or the OLED 10A or the OLED 10B) of the imaging device 901 is configured to switchably emit colored light, NBI light, and structured light toward outside of the distal end 901b. In some embodiments, the OLED 10 (or the OLED 10A or the OLED 10B) of the imaging device 901 emits light along a light emitting direction DR1. In some embodiments, the working channel outlet is located on a surface of the distal end 901b facing the light emitting direction DR1 for delivering/aspirating liquid. In some embodiments, the distal end 901b does not include an LED or a color filter. In some embodiments, the distal end 901b does not include an optical fiber.

In some embodiments, the bending portion 901e is connected to the distal end 901b. The bending portion 901e is movable, such that the lens end (i.e., the distal end 901b) articulates upward or downward in response to operation of the control lever. In some embodiments, the bending portion 901e includes a hollow conduit 901e1. In some embodiments, the hollow conduit 901e1 of the bending portion 901e communicates with the hollow conduit 901a1 of the holding portion 901a. In some embodiments, the wire structure 901R passes through the hollow conduit 901e1 of the bending portion 901e and the hollow conduit 901a1 of the holding portion 901a to transmit a control signal to the OLED to switch between emitting the colored light, the NBI light and the structured light. In some embodiments, in a planar light source mode, all pixels are lit synchronously to realize uniform planar illumination for emitting white light or color light, in a structured light mode, only pixels of a predetermined pattern (for example, stripe pixels) are lit according to a predetermined stripe pattern to form a high frequency patterned grating (for example, a stripe grating), and in a multicolor/narrow band mode, single-color or narrow band light emission is implemented by selecting the corresponding pixel array, wherein all switching times between the modes are less than 5 ms. In some embodiments, the wire structure 901R passes through the hollow conduit 901e1 of the bending portion 901e and the hollow conduit 901a1 of the holding portion 901a to transmit power to the organic light emitting component and the image sensor. In some embodiments, the wire structure 901R passes through the hollow conduit 901e1 of the bending portion 901e and the hollow conduit 901a1 of the holding portion 901a to transmit image information captured by the image sensor to the device 901c outside the holding portion 901a. In some embodiments, the wire structure 901R includes a wire (or a signal line) for transmitting the control signal, a wire (or an image transmission line) for transmitting the image information, and a wire (or a power line) for transmitting the power.

In some embodiments, the OLED inside the distal end 901b is directly deposited on a metal or glass substrate 100 and connected to the wire structure 901R through metal electrodes 215, 216, 225, and 235. In some embodiments, the substrate 100 of the OLED is selected from a thin glass with low thermal expansion or a flexible polyimide film to ensure dimensional stability even when the bending portion 901e is bent. In some embodiments, the electrodes 215, 216, 225, and 235 use aluminum or silver films, and grooves are reserved at electrode edges for the connection with the wire structure 901R.

In some embodiments, the device 901c may include a processor and software for receiving and processing the image information. In some embodiments, the device 901c may include a processor for providing the control signal. In some embodiments, the device 901c may include a power supply.

Conventionally, LEDs together with filters are commonly used as the light sources of endoscope probes, but the combination of LEDs and filters cannot achieve the effect of high pixel density. In contrast, according to some embodiments of the present application, using OLEDs as the light sources can achieve the effect of high pixel density. Compared to conventional LEDs (pixel size ≥ 100 µm), the pixel size of OLEDs is only about 10 µm, which enables a light source resolution of >10,000 ppi at the probe ends, and thus the detail of structured light patterns and the accuracy of 3D measurement are significantly improved. Compared to conventional optical fiber bundles (for example, 30 optical fibers) that can produce only low-resolution single-color gratings, the pixel array of OLEDs can directly output micron-level stripes, and thus the grating frequency is increased by more than 10 times, and the accuracy and speed of 3D measurement are significantly improved.

Furthermore, according to some embodiments of the present application, the operating voltage of an OLED is about 3 V to 5 V, the power consumption of the OLED is only 1/3 to 1/4 of that of an LED with the same brightness (about 10mW/cm²), and the heat generation caused by operating power is less than 5°C, and these allow long term operation within a narrow space with a probe volume less than 2 mm (hollow conduits 901a1 and 901e1) without causing thermal damage to the device materials, so that the high temperature limitation when LEDs are placed at the probe ends is overcome, and thus reduced power consumption and improved heat dissipation are achieved.

Also, according to some embodiments of the present application, by sending the control signal to the OLED(s) through the wire structure 901R, instantaneous switching between a planar light source, striped structured light, and a multicolor/narrow band light source can be accomplished without replacing optical fibers or filters or requiring external filters or multiple LEDs, a single OLED can complete the rapid switching between white light, colored light, narrow band light, and structured light within the same probe, all light source functions are implemented within a single OLED, and the optical path has only one light output, the optical path design is significantly simplified, and the system size and complexity are greatly reduced, these allow easy integration with existing flexible endoscopes (diameter ≤ 2.5 mm). In particular, the narrow band light source can provide high contrast images at the heme absorption peaks (415 nm and 530 nm) of blood vessels or mucosal tissues, and the visualization of early lesions is further enhanced.

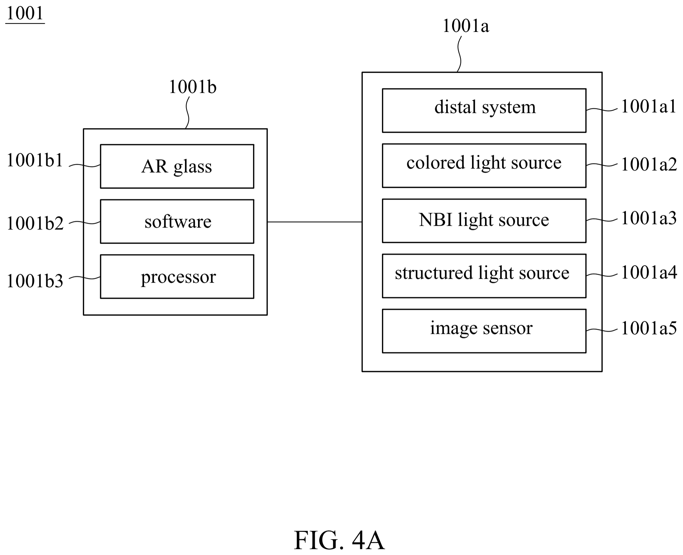

FIG. 4A is a diagram exemplarily showing an imaging system 1001. In some embodiments, FIG. 4A shows an arrangement of components of the imaging device 1001. In some embodiments, the imaging device 1001 may include the imaging device 901. In some embodiments, the imaging system 1001 is a single-use or disposable imaging system, such as a single-use endoscope.

In some embodiments, the imaging device 1001 may include a single-use component 1001a and a reusable component 1001b. In some embodiments, the single-use component 1001a may include a distal system 1001a1, a colored light source 1001a2, an NBI light source 1001a3, a structured light source 1001a4, and an image sensor 1001a5. In some embodiments, the distal system 1001a1 may include the colored light source 1001a2, the NBI light source 1001a3, the structured light source 100a4, and the image sensor 1005 which is configured to capture the image information. In some embodiments, the distal system 1001a1 does not include an optical fiber, an LED, or a color filter. In some embodiments, the reusable component 1001b may include an AR glasses 1001b1, a swarm algorithm software 1001b2, and a processor 1001b3.

In some embodiments, the distal system 1001a1 is configured to capture image information of an object under test and transmit the image information to the processor 1001b3. In some embodiments, the colored light source 1001a2 may be a 5 in 1 colored light source. In some embodiments, the colored light source 1001a2 may include an OLED 10, 10A, or 10B as described above. In some embodiments, the NBI light source 1001a3 may include an OLED 10, 10A, or 10B as described above, with a light emitting wavelength of about 415 nm and/or about 530 nm. In some embodiments, the structured light source 100a4 may include an OLED 10, 10A, or 10B as described above, with a light emitting wavelength in a range from ultraviolet to near-infrared light. In some embodiments, the image sensor 1001a5 may include a CMOS image sensor (CIS).

In some embodiments, the AR glasses 1001b1may include an OLED 10, 10A, or 10B as described above. In some embodiments, the processor 1001b3 transmit the control signal to control the OLED 10, 10A, or 10B to switch between emitting the colored light, the NBI light, and the structured light, so as to implement the colored light source 1001a2, the NBI light source 1001a3, and the structured light source 100a4 of the distal system 1001a1. In other words, by the control of the processor 1001b3, the OLED 10, 10A, or 10B may include or have all three light sources, i.e., the colored light source 1001a2, the NBI light source 1001a3, and the structured light source 1001a4. In some embodiments, the processor 1001b3 is configured to process the captured image information to generate an image of an appearance of the object. In some embodiments, the processor 1001b3 is configured to process the captured image information to generate a 3D structural simulation result of the object. In some embodiments, the processor 1001b3 may include a CPU and/or a GPU. In some embodiments, the AR glasses 1001b1 is configured to receive the image of the appearance of the object, so as to present a real-time image in a display area of the AR glasses 1001b1. In some embodiments, the AR glasses 1001b1 is configured to receive the 3D structural simulation result of the object, and after processed by the processor 1001b3 using a swarm algorithm, to present a real-time image in the display area of the AR glasses 1001b1. In some embodiments, the AR glasses 1001b1 is configured to receive the image of the appearance and the 3D structural simulation result of the object, and after processed by the processor 1001b3 using a swarm algorithm, to present a real-time image in the display area of the AR glasses 1001b1. In some embodiments, the AR glasses 1001b1 may include the processor 1001b3.

According to some embodiments of the present application, the acquired high-resolution 3D appearance is projected in real time onto the AR glasses 1001b1 by the processor 1001b3, and "naked-eye 3D navigation" of the surgical site can be achieved. Conventional LED light sources cannot provide narrow band high-resolution images, and therefore, the combination the AR glasses 1001b1 has significant additional technical effects, and the intuitiveness and accuracy of surgical positioning can be improved.

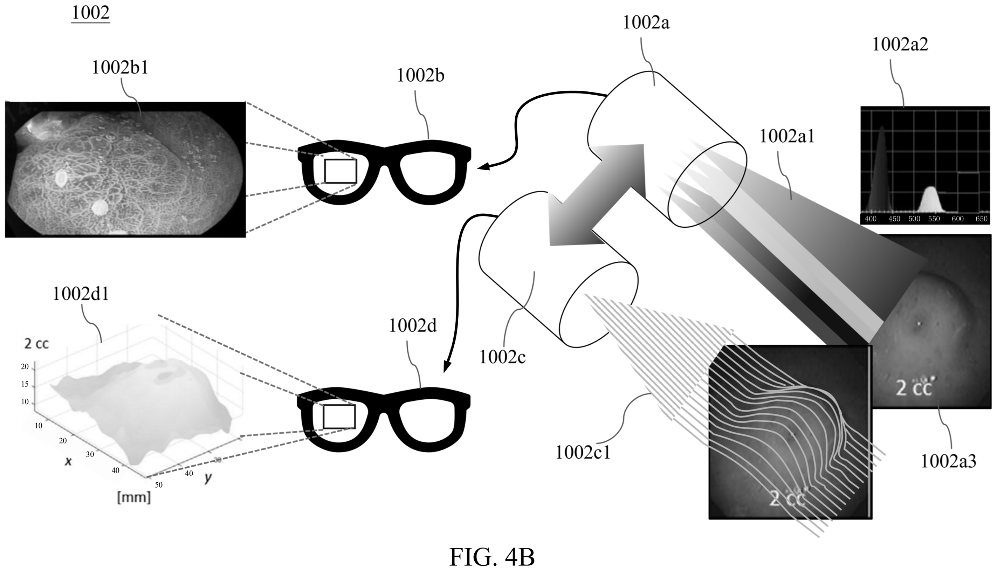

FIG. 4B is a diagram exemplarily showing an imaging system 1002.In some embodiments, the imaging device 1002 may include the imaging device 901. In some embodiments, the imaging device 1002 may include components of the imaging device 1001 as described above. In some embodiments, the imaging device 1002 may include distal systems 1002a and 1002c and AR glasses 1002b and 1002d. The distal systems 1002a and 1002c are, for example, probes of the imaging system 1002 or an endoscope configured to extend into a living organism for detection. In some embodiments, the distal systems 1002a and 1002c may include the distal system 1001a1 as described above. In some embodiments, the AR glasses 1002b and 1002d may include the AR glasses 1001b1 as described above.

In some embodiments, the distal system 1002a may include an OLED 10, 10A, or 10B as described above, which can be used as a colored light source and a NBI light source for providing colored light and NBI light. In some embodiments, the distal systems 1002a and 1002c may provide colored light 1002a1. In some embodiments, the distal systems 1002a and 1002c may provide NBI light as shown in the spectrum 1002a2.When the distal system 1002a illuminates the object under test with white light rather than using a colored light source or a NBI light source, the distal system 1002a can capture only a blurred appearance, as shown in the photo 1002a3, and cannot present a detailed image. When the distal system 1002a illuminates the object under test with the colored light 1002a1 or NBI light, the captured image information is transmitted to the AR glass 1002b and processed by its processor using swarm algorithm software, and a detailed image as shown in the photo 1002b1 can be obtained. In some embodiments, the switch between the colored light source and the NBI light source can be done by selecting different pixel sub-arrays (corresponding to the light emitting units 101, 102, and 103) or switching different organic emissive layers (corresponding to the organic emission layers 264 of the light emitting units 101, 102, and 103), and multi-color (red, green, and blue) combinations can be realized in a planar light source mode, or the NBI light source can be implemented by lighting only pixels in narrow band at 415 nm or 530 nm. In some embodiments, a switching time between the modes is less than 5 ms, the power consumption is kept within a range from 10 mW to 15 mW, the temperature rise at the probe end does not exceed 2°C, and real-time and seamless light source switching can be achieved during surgery. According to some embodiments of the present application, by illuminating the object under test with the NBI light to capture the image information, the contrast of the image can be improved, and thus the noise ratio can be reduced.

In some embodiments, the distal system 1002c may include an OLED 10, 10A, or 10B as described above, which can be used as a structured light source for providing structured light 1002c1. The structured light 1002c1 may have a light emitting wavelength in a range from ultraviolet to near-infrared light. In some embodiments, the structured light source is configured to illuminate the object under test with the structured light 1002c1 having a light pattern, and the image information of the deformation degree of the light pattern is transmitted to the AR glasses 1002d and processed by its processor using swarm algorithm software, and a 3D structural simulation result as shown in the image 1002d1 can be obtained. In some embodiments, the distal systems 1002a and 1002c can switch with each other, so as to obtain the image of the appearance or the 3D structural simulation result of the object under test.

A current approach is using the combination of LEDs and filters as the colored light sources of the endoscopes, but the combination of LEDs and filters cannot achieve the effect of high pixel density and also cannot form structured light with fine patterns, optical fibers are still required to transmit light to the probe ends (that is, to transmit the light from the device side of the imaging system 1002 to the light source on the probe), and the size of the optical fibers also limits how small the probe can be made. Another current approach is arranging white light sources on the probes, where the optical fibers are not needed for the light transmission, but white light provides only an illumination effect and cannot provide a corresponding light source based on the absorption spectrum of the object under test, and therefore, the image display performance is poor and the resolution is also lower.

According to some embodiments of the present application, using OLEDs as the light sources can achieve the effect of high pixel density, the power consumption can be further reduced, and the heat dissipation issue can be addressed. Furthermore, the OLED(s) can provide a colored light source, an NBI light source, and a structured light source, and, by using the AR glasses with the OLED(s), a high resolution photo and a 3D simulation result can be obtained, and a high resolution real-time image and a 3D simulation real-time image are presented in the display area of the AR glasses, and thus it is beneficial for reducing surgical time.

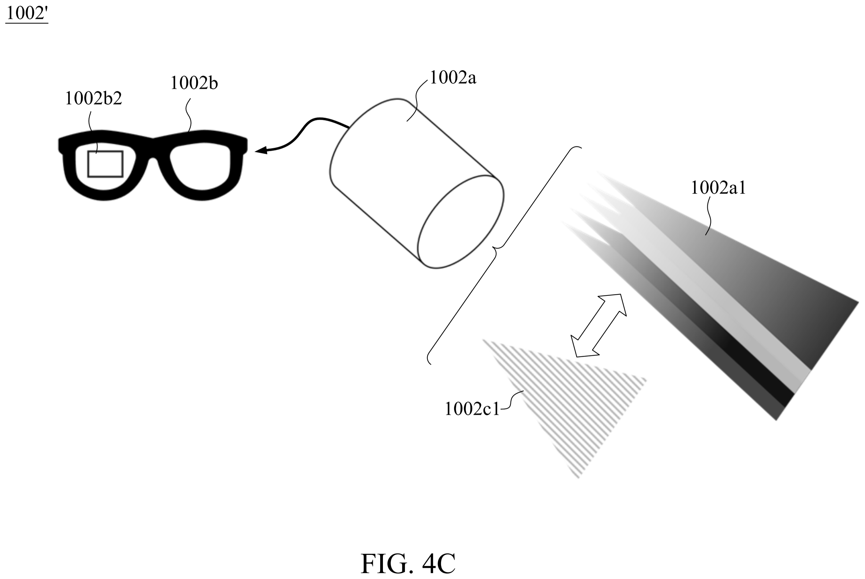

FIG. 4C is a diagram exemplarily showing an imaging system 1002'. In some embodiments, the imaging device 1002' may include the imaging device 901. In some embodiments, the imaging device 1002' may include components of the imaging device 1001 as described above. In some embodiments, the imaging device 1002' may include the distal system 1002a and the AR glasses 1002b. The distal system 1002a is, for example, a probe of the imaging system 1002' or an endoscope configured to extend into a living organism for detection. In some embodiments, the distal system 1002a may include the distal system 1001a1 as described above. In some embodiments, the AR glasses 1002b may include the AR glasses 1001b1 as described above.

In some embodiments, the distal system 1002a may include an OLED 10, 10A, or 10B as described above, which is configured to switchably emit the colored light, the NBI light, and the structured light. In some embodiments, the distal system 1002a may provide the colored light 1002a1, the NBI light as shown in the spectrum 1002a2, and the structured light 1002c1. When the distal system 1002a illuminates the object under test with the colored light 1002a1 or the NBI light, the captured image information is transmitted to the AR glass 1002b and processed by its processor using swarm algorithm software, and a detailed image as shown in the photo 1002b1 of FIG. 4B can be obtained. When the distal system 1002a illuminates the object under test with the structured light 1002c1, the image information of the deformation degree of the light pattern is transmitted to the AR glasses 1002b and processed by its processor using swarm algorithm software, and a 3D structural simulation result as shown in the image 1002d1 of FIG. 4B can be obtained.

In some embodiments, the AR glasses 1002b is configured to receive the image of the appearance (for example, the detailed image as shown in the photo 1002b1 of FIG. 4B) and the 3D structural simulation result (for example, the 3D structural simulation result as shown in the image 1002d1 of FIG. 4B) of the object, and after processed by the processor 1001b3 using a swarm algorithm, to present a real-time image 1002b2 in the display area of the AR glasses 1002b.

According to some embodiments of the present application, by using the OLED(s) of the distal system 1002a, the colored light source, the NBI light source, and the structured light source can be switched and provided, and, by using the AR glasses with the OLED(s), a high resolution detailed image and a 3D simulation result of the object can be obtained, and the high resolution real-time image 1002b2 and the 3D simulation real-time image 1002b2 can be presented in the display area of the AR glasses, and thus it is beneficial for reducing surgical time.

Furthermore, according to some embodiments of the present application, rapid switching between the planar light source and a structured light can be accomplished without having to mechanically move optical components or change filters, and thus the need of real-time surgical navigation can be satisfied. In other words, a surgeon can switch among the three modes of "colored planar light source - narrow band light source - structured light 3D measurement" simply through a handheld control panel or voice commands, and the cumbersome steps of replacing optical fibers, filters, or additional light sources are eliminated. Through collaboration with the AR glasses, real-time 3D navigation of the surgical site can be achieved, and the safety and accuracy of surgical procedures are significantly enhanced.

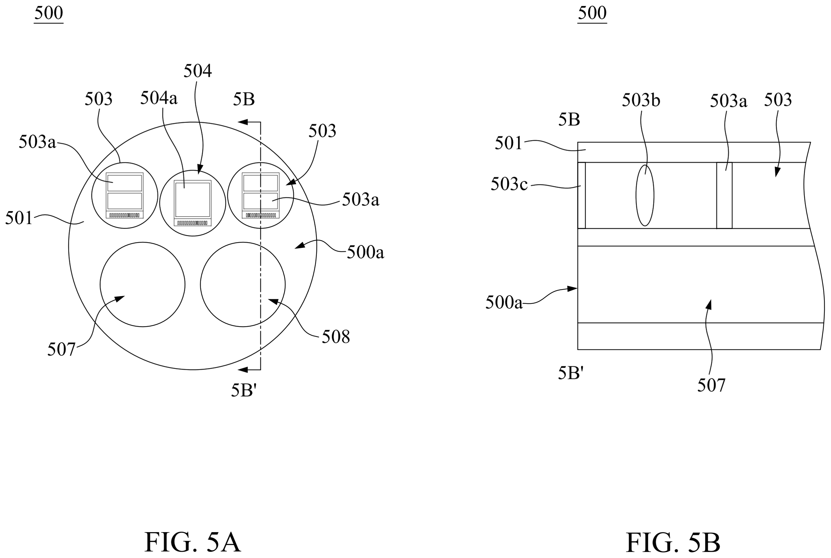

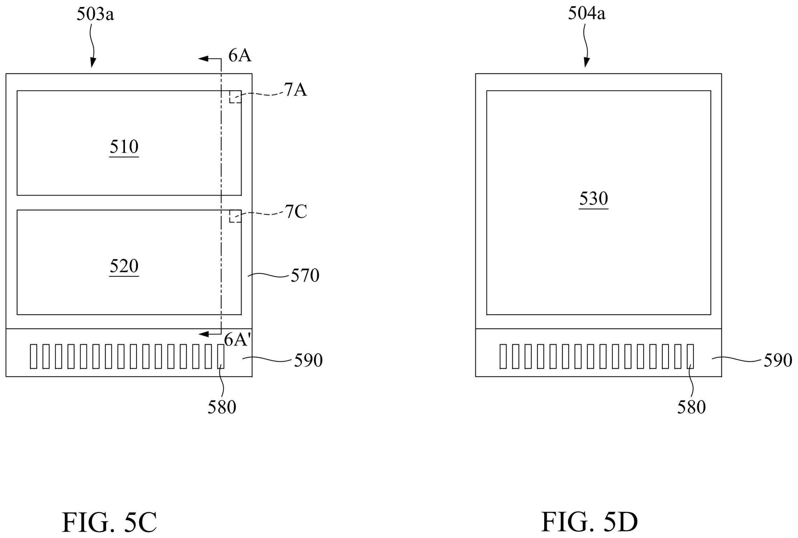

FIG. 5A is a diagram exemplarily showing a distal system 500.FIG. 5B is a cross sectional view exemplarily showing the distal system 500.FIG. 5C is a top view exemplarily showing a light emitting device 503a. FIG. 5D is a top view exemplarily showing an image sensor structure 504a. In some embodiments, FIG. 5A is a top view exemplarily showing the distal system 500 of an imaging system, and FIG. 5B is a cross sectional view exemplarily taken along line 5B-5B' in FIG. 5A. In some embodiments, FIG. 5C is a top view exemplarily showing a light emitting device 503a of the distal system 500, and FIG. 5D is a top view exemplarily showing an image sensor structure 504a of the distal system 500.In some embodiments, the distal system 500 may be or include the distal end 901b and/or the distal system 1001a1 as described above. In some embodiments, the distal end 901b of the imaging device 901 shown in FIG. 3 may be or include the distal system 500.In some embodiments, the distal system 1001a1 of the imaging system 1001 shown in FIG. 4 may be or include the distal system 500.In some embodiments, the distal system 500 is the distal system of a single-use or disposable imaging system, such as the distal system of a single-use endoscope.

Referring to FIG. 5A and FIG. 5B, in some embodiments, the distal system 500 is electrically connected to the processor of the imaging system. In some embodiments, the distal system 500 includes the light emitting device 503a and the image sensor structure 504a. In some embodiments, the distal system 500 further includes a housing 501, a hollow conduit 503 (also referred to as a first hollow conduit), and a hollow conduit 504 (also referred to as a second hollow conduit). In some embodiments, the light emitting device 503a is disposed in the hollow conduit 503, and the image sensor structure 504a is disposed in the hollow conduit 504. In some embodiments, the hollow conduits 503 and 504 respectively fix the substrates of the light emitting device 503a and the image sensor structure 504a, and lead out wires connected to the long wire structure 901R, and the wire structure passes through the hollow conduits of the bending portion 901e and the holding portion 901a, and finally connects to the device 901c outside the holding portion 901a to transmit power and a signal for the light emitting device 503a and the image sensor structure 504a.

In some embodiments, the distal system 500 further includes a transparent protection plate 503c covering an opening of the hollow conduit 503 and a lens 503b disposed between the transparent protection plate 503c and the light emitting device 503a. In some embodiments, the lens 503b is configured to focus light emitted from the light emitting device 503a. The position of the lens 503b can be determined according to its focal length and can be adjusted in conjunction with a motor and a mechanism to change the focal length or depth of field. The transparent protection plate 503c may include a cover glass, which can be used to prevent moisture from entering the hollow conduit.

In some embodiments, the distal system 500 further includes working channels 507 and 508 for delivering/aspirating fluids or inserting other endoscopic instruments, and outlets of the working channels 507 and 508 are located on a surface 500a of the distal system 500 facing a light emitting direction. In some embodiments, the working channels 507 and 508 may include a surgical opening and a water outlet, respectively.

In some embodiments, the light emitting device 503a is configured to switchably emit colored light having two or more colors, NBI light having a FWHM less than 30 nm, and structured light having a predetermined pattern. In some embodiments, each of the colored light and the structured light 1002c1 may have a light emitting wavelength in a range from 200 nm to 3,000 nm.

Referring to FIG. 5C and FIG. 5D, in some embodiments, the light emitting device 503a includes an OLED, a micro-LED, a QLED, or any combination thereof. In some embodiments, the OLED may include the OLED 10, 10A, or 10B as described above, and for example, the OLED may include the electrode, the semi-reflective surface (or the first reflective surface), the fully reflective surface (or the second reflective surface), and the organic light emitting layer, wherein the fully reflective surface (or the second reflective surface) has a reflectance greater than a reflectance of the semi-reflective surface (or the first reflective surface), the organic light emitting layer is disposed above the electrode between the semi-reflective surface and the fully reflective surface, and the distance between the semi-reflective surface and the fully reflective surface defines a micro resonant cavity.

In some embodiments, the light emitting device 503a includes a substrate 590, a micro-LED array 510 disposed on the substrate 590, and an OLED array 520 disposed on the substrate 590.The substrate 590 may be a silicon wafer or a glass wafer. In some embodiments, the micro-LED array 510 includes said micro-LED, and the OLED array 520 includes said OLED. The OLED array 520 may be or include a micro organic light emitting device array (micro-OLED array). In some embodiments, light emitted from the micro-LED array 510 having one or more colors is used to compensate for luminous efficiency of light emitted from a plurality of OLEDs of the OLED array 520 having one or more colors.

In some embodiments, the light emitting device 503a is a hybrid light source, the hybrid arrangement of the micro-LED array 510 and the OLED array 520 is provided for mutual color compensation, and in particular, the blue light luminous efficiency of the OLEDs is relatively low, the blue light of the micro-LEDs can be used to assist or enhance the light emission, and similarly, the micro-LEDs can also assist the purple light that is difficult to achieved by the OLED. The light emission mode of the light emitting device 503a can be switched instantaneously under the control of the processor 1001b3, and it can emit the colored light, the NBI light having the FWHM less than 30 nm, and the structured light having the predetermined pattern. The design with the FWHM less than 30 nm is intended to make the light more concentrated and purer, so as to achieve narrow band emission, and thus higher detection sensitivity can be obtained. The light emitting device 503a can also directly output directly output micron-level stripes as the structured light, and the accuracy of 3D measurement can be significantly improved.

In some embodiments, the light emitting device 503a further includes a dam structure 570.In some embodiments, the dam structure 570 surrounds the micro-LED array 510 and the OLED array 520.In some embodiments, the dam structure 570 may include the protrusions 310 as described above. In some embodiments, the light emitting device 503a further includes conductive pads 580 disposed on the substrate 590.In some embodiments, the conductive pads 580 may be electrically connected to the micro-LED array 510, the OLED array 520, and the wire structure 901R as described above through the substrate 590.By arranging the dam structure 570, the micro-LED array 510 and the OLED array 520 can be protected from the influence of the external environment, and optical optimization can be performed.

In some embodiments, the image sensor structure 504a includes a substrate 590, an image sensor array 530 disposed on the substrate 590, and conductive pads 580 disposed on the substrate 590.In some embodiments, the conductive pads 580 of the image sensor structure 504a may be electrically connected to the image sensor array 530 and the wire structure 901R as described above through the substrate 590.The image sensor array 530 may include a plurality of image sensors (also referred to as imagers), each of which may include a CMOS image sensor (CIS). The image sensors may include cameras.

In some embodiments, the image sensors (or the imagers) and multicolor light sources may be integrated or disposed on the same substrate. In some embodiments, as shown in FIG. 5A, the micro-LED array 510, the OLED array 520, and the conductive pads 580 are disposed on the same substrate 590, and the substrate 590 may include a silicon wafer or a glass wafer. In some embodiments, as shown in FIG. 5A, two light emitting device 503a are symmetrically distributed on two sides of the image sensor structure 504a to achieve a more uniform illumination effect. The light emitting device 503a and the image sensor structure 504a share the conductive pads 580 on the substrate 590, and connected to the power supply and a signal processing unit at the back end through the wire structure 901R.

According to some embodiments of the present application, the distal system 500 is the probe portion for a single-use imaging system (for example, a single-use endoscope, and the luminescence function, imaging function, and the working signal channels are integrated into a compact structure to achieve advantages of high safety, low cost, and accurate diagnosis.

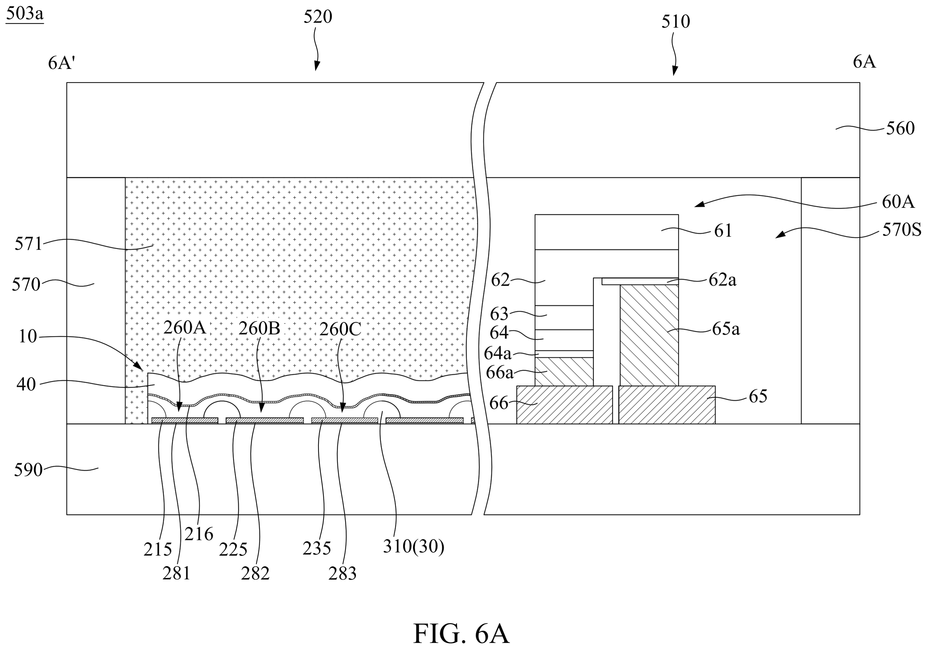

FIG. 6A is a cross sectional view exemplarily showing the light emitting device 503a. In some embodiments, FIG. 6A is a cross sectional view exemplarily taken along line 6A-6A' in FIG. 5C.

In some embodiments, the light emitting device 503a includes the substrate 590, the micro-LED array 510, the OLED array 520, a transparent cover plate 560, the dam structure 570, a transparent encapsulation layer 571, and the conductive pads 580.In some embodiments, the micro-LED array 510 includes a micro-LED 60A, and the OLED array 520 includes the OLED 10.In some embodiments, the transparent encapsulation layer 571 covers the OLED array 520 and exposes the micro-LED array 510.In some embodiments, the micro-LED 60A is exposed in space 570S defined by the dam structure 570 and the transparent cover plate 560.

In some embodiments, the OLED 10 includes the structure of the OLED 10, 10A, or 10B as described above, for example, the OLED 10 includes at least the electrodes 215, 225, and 235, the organic light emitting layers 260A, 260B, and 260C, the reflective layers 281, 282, and 283, the spacer structure 30, and the covering layer 40.

In some embodiments, the micro-LED 60A includes a substrate 61, an n-type GaN layer 62, a connecting layer 62a, a light emitting layer 63, a p-type GaN layer 64, a connecting layer 64a, an n-type electrode 65, a connecting layer 65a, a p-type electrode 66, and a connecting layer 66a. In some embodiments, the light emitting layer 63 includes a multiple quantum well (MQW) structure. In some embodiments, the substrate 61 includes a ceramic substrate, such as aluminum oxide. In some embodiments, the connecting layer 62a and the connecting layer 64a includes a solder material. In some embodiments, the n-type electrode 65 and the p-type electrode 66 include a metal material, such as AuSn alloy. In some embodiments, the connecting layer 65a and the connecting layer 66a includes a metal material, such as Au.

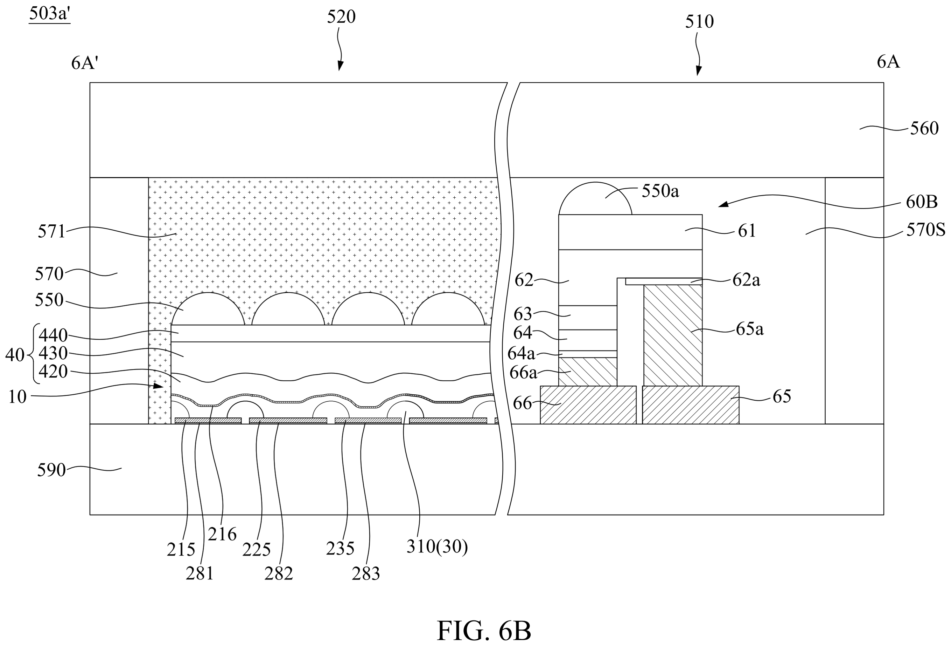

FIG. 6B is a cross sectional view exemplarily showing a light emitting device 503a'. In some embodiments, FIG. 6B is a cross sectional view exemplarily taken along line 6A-6A' in FIG. 5C.

In some embodiments, the light emitting device 503a' further includes a microlens structure 550 (also referred to as a first microlens structure) disposed on a light emitting surface of the OLED 10 and a microlens structure 550a (also referred to as a second microlens structure) disposed on a light emitting surface of the micro-LED 60B. In some embodiments, the micro-LED 60B includes the microlens structure 550a.

In some embodiments, each of the microlens structures 550 and 550a may include a plurality of microlenses. In some embodiments, a microlens is disposed on a light emitting unit. For example, in the case where an OLED 10 includes three light emitting units (pixels), a microlens is disposed on each light emitting unit. In some embodiments, the microlens structures 550 and 550a are configured to collimate emitted light and improve the light emission angle. In some embodiments, only one microlens is disposed on the light emitting unit of each micro-LED 60B, which means that each micro-LED 60B emits only single-color light.

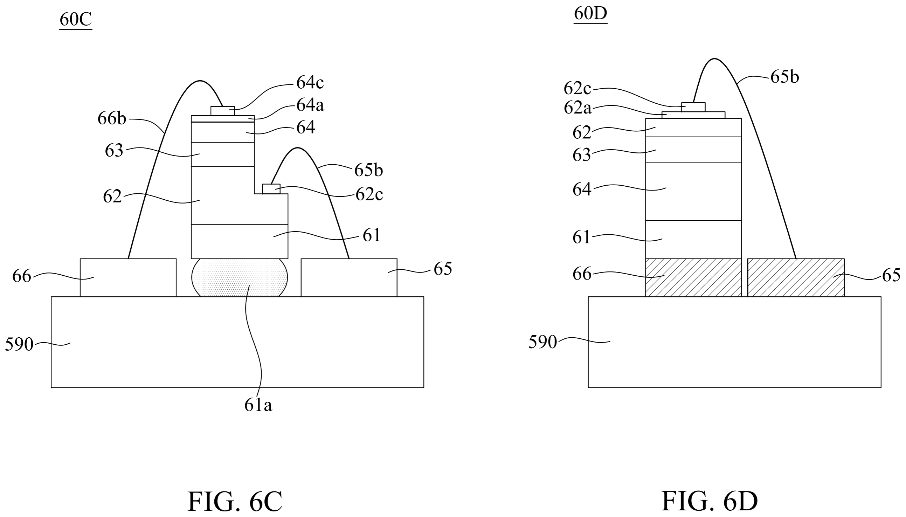

FIG. 6C is a cross sectional view exemplarily showing a micro-LED 60C. In some embodiments, each of the micro-LED array described herein may include a micro-LED 60C as shown in FIG. 6C.

In some embodiments, the micro-LED 60C includes a substrate 61, a connecting layer 61a, an n-type GaN layer 62, a conductive pad 62c, a light emitting layer 63, a p-type GaN layer 64, a connecting layer 64a, a conductive pad 64c, an n-type electrode 65, a connecting wire 65b, a p-type electrode 66, and a connecting wire 66b. In some embodiments, the connecting layer 61a and the connecting layer 64a includes a solder material.

FIG. 6D is a cross sectional view exemplarily showing a micro-LED 60D. In some embodiments, each of the micro-LED array described herein may include a micro-LED 60D as shown in FIG. 6D.

In some embodiments, the micro-LED 60D includes a substrate 61, an n-type GaN layer 62, a connecting layer 62a, a conductive pad 62c, a light emitting layer 63, a p-type GaN layer 64, an n-type electrode 65, a connecting wire 65b, and a p-type electrode 66.

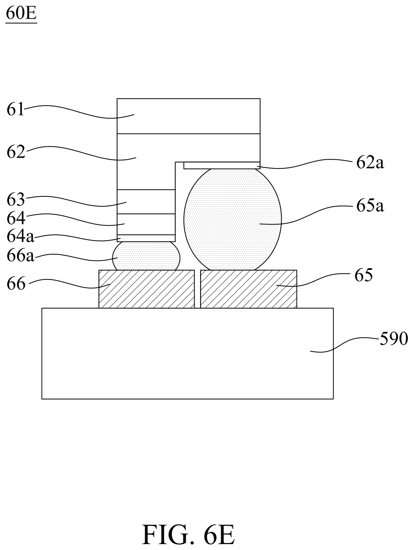

FIG. 6E is a cross sectional view exemplarily showing a micro-LED 60E. In some embodiments, each of the micro-LED array described herein may include a micro-LED 60E as shown in FIG. 6E.

In some embodiments, the micro-LED 60A includes a substrate 61, an n-type GaN layer 62, a connecting layer 62a, a light emitting layer 63, a p-type GaN layer 64, a connecting layer 64a, an n-type electrode 65, a connecting wire 65b, a p-type electrode 66, and a connecting layer 66a.

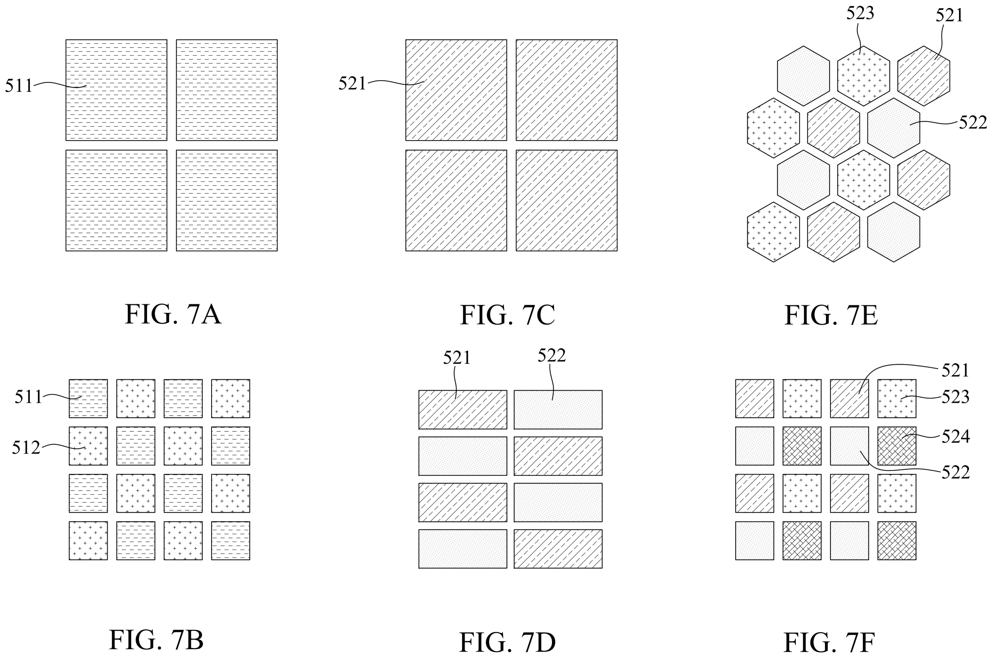

FIG. 7A is a top view exemplarily showing a portion 7A of the micro-LED array 510.In some embodiments, the micro-LED array 510 includes an array constituted by a plurality of light emitting units 511 of single-color micro-LEDs.

FIG. 7B is a top view exemplarily showing the portion 7A of the micro-LED array 510.In some embodiments, the micro-LED array 510 includes an array constituted by a plurality of light emitting units 511 of single-color micro-LEDs and a plurality of light emitting units 512 of single-color micro-LEDs The light emitting units 511 and the light emitting units 512 constitute a mixed two-color micro-LED array 510.In some embodiments, the light emitting units 511 emit purple light, the light emitting units 512 emit blue light, and the blue light emitting units 512 can help enhance the relatively insufficient luminous efficiency of the purple light emitting units 511.

FIG. 7C is a top view exemplarily showing a portion 7C of the OLED array 520.In some embodiments, the OLED array 520 includes an array constituted by a plurality of light emitting units 521 of single-color OLEDs.

FIG. 7D is a top view exemplarily showing the portion 7C of the OLED array 520.In some embodiments, the OLED array 520 includes an array constituted by a plurality of light emitting units 521 of single-color OLEDs and a plurality of light emitting units 522 of single-color OLEDs. The light emitting units 521 and the light emitting units 522 constitute a mixed two-color micro-LED array 520.In some embodiments, the light emitting layer 521 emit green light, and the light emitting layer 522 emit red light.

FIG. 7E is a top view exemplarily showing the portion 7C of the OLED array 520.In some embodiments, the OLED array 520 includes an array constituted by a plurality of light emitting units 521 of single-color OLEDs, a plurality of light emitting units 522 of single-color OLEDs, and a plurality of light emitting units 523 of single-color OLEDs. The light emitting units 521, the light emitting units 522, and the light emitting units 523 constitute a mixed three-color micro-LED array 520.In some embodiments, the light emitting layer 521 emit green light, the light emitting layer 522 emit red light, and the light emitting layer 523 emit blue light. In some embodiments, the respective light emitting units or the respective OLEDs may have different shapes, and may be formed by patterning techniques, such as dry etching or wet etching.

FIG. 7F is a top view exemplarily showing the portion 7C of the OLED array 520.In some embodiments, the OLED array 520 includes an array constituted by a plurality of light emitting units 521 of single-color OLEDs, a plurality of light emitting units 522 of single-color OLEDs, a plurality of light emitting units 523 of single-color OLEDs, and a plurality of light emitting units 524 of single-color OLEDs. The light emitting units 521, the light emitting units 522, the light emitting units 523, and the light emitting units 524 constitute a mixed four-color micro-LED array 520.In some embodiments, the light emitting layer 521 emit green light, the light emitting layer 522 emit red light, the light emitting layer 523 emit blue light, and the light emitting layer 524 emit yellow light.

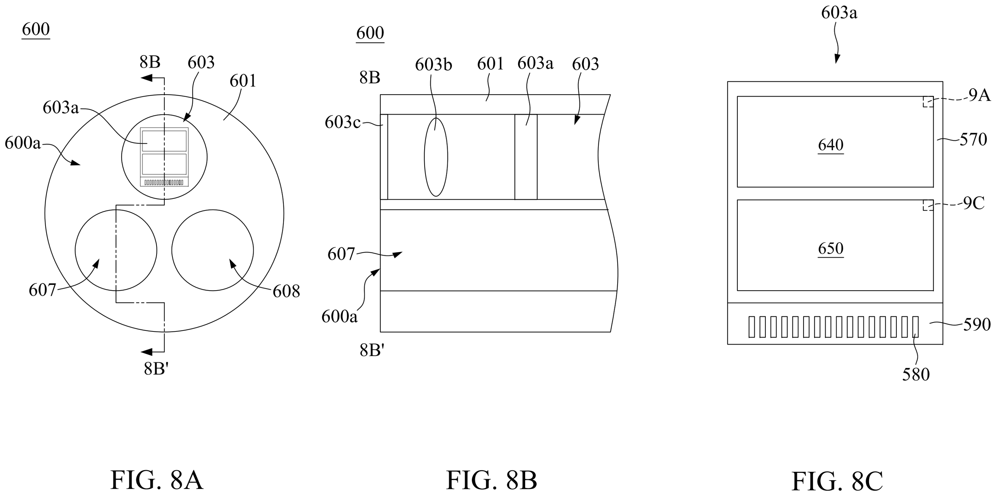

FIG. 8A is a diagram exemplarily showing a distal system 600.FIG. 8B is a cross sectional view exemplarily showing the distal system 600.FIG. 8C is a top view exemplarily showing a light emitting device 603a. In some embodiments, FIG. 8A is a top view exemplarily showing the distal system 600 of an imaging system, and FIG. 8B is a cross sectional view exemplarily taken along line 8B-8B' in FIG. 8A. In some embodiments, FIG. 8C is a top view exemplarily showing the light emitting device 603a of the distal system 600.In some embodiments, the distal system 600 may be or include the distal end 901b and/or the distal system 1001a1 as described above. In some embodiments, the distal end 901b of the imaging device 901 shown in FIG. 3 may be or include the distal system 600.In some embodiments, the distal system 1001a1 of the imaging system 1001 shown in FIG. 4 may be or include the distal system 600.In some embodiments, the distal system 600 is the distal system of a single-use or disposable imaging system, such as the distal system of a single-use endoscope.

Referring to FIG. 8A and FIG. 8B, in some embodiments, the distal system 600 is electrically connected to the processor of the imaging system. In some embodiments, the distal system 600 includes the light emitting device 603a, which is integrated with an image sensor structure. In some embodiments, the light emitting device 603a includes an integrated structure of an OLED, a micro-LED, and the image sensor structure (or an imager). In some embodiments, the integrated structure of the light emitting device 603a includes the image sensor structure (or the imager), multicolor light sources, and the conductive pads, wherein the multicolor light sources may include a micro-LED array and an OLED array, the image sensor structure (or the imager)) includes an image sensor array, which is integrated into the micro-LED array and the OLED array.

In some embodiments, the distal system 600 further includes a housing 601 and a hollow conduit 603. In some embodiments, the light emitting device 603a is disposed in the hollow conduit 603. In some embodiments, the distal system 600 further includes a transparent protection plate 603c covering an opening of the hollow conduit 603 and a lens 603b disposed between the transparent protection plate 603c and the light emitting device 603a. The transparent protection plate 603c may include a cover glass.

In some embodiments, the distal system 600 further includes working channels 607 and 608 for delivering/aspirating fluids or inserting other endoscopic instruments, and outlets of the working channels 607 and 608 are located on a surface 600a of the distal system 600 facing a light emitting direction. In some embodiments, the working channels 607 and 608 may include a surgical opening and a water outlet, respectively.

In some embodiments, the light emitting device 603a is configured to switchably emit colored light having two or more colors, NBI light having a FWHM less than 30 nm, and structured light having a predetermined pattern.

Referring to FIG. 8C, in some embodiments, the light emitting device 603a includes an OLED, a micro-LED, a QLED, or any combination thereof. In some embodiments, the OLED may include the OLED 10, 10A, or 10B as described above.

In some embodiments, the light emitting device 603a includes a substrate 590, a micro-LED array 640 disposed on the substrate 590, an OLED array 650 disposed on the substrate 590, the dam structure 570, and the conductive pads 580.In some embodiments, the micro-LED array 640 is also referred to as a micro-LED integrated array, and the OLED array 650 is also referred to as an OLED integrated array. The OLED array 650 may be or include a micro-OLED array. In some embodiments, the image sensor structure of the light emitting device 603a includes a plurality of image sensors (also referred to as first image sensors) integrated into the OLED array and a plurality of image sensors (also referred to as second image sensors) integrated into the micro-LED array, and the first image sensors and the second image sensors are all disposed on the substrate 590.In some embodiments, the micro-LED array 640 includes micro-LEDs and the plurality of image sensors integrated into the micro-LED array, and the OLED array 650 includes OLEDs and the plurality of image sensors integrated into the OLED array, In some embodiments, light emitted from the micro-LED array 640 having one or more colors is used to compensate for luminous efficiency of light emitted from a plurality of OLEDs of the OLED array 650 having one or more colors.

According to some embodiments of the present application, the light emitting device 603a integrated with the image sensor structure includes both of the multicolor light sources (micro-LED array and micro-OLED array) and the image sensor structure, which are all integrated onto the same substrate 590, such an integrated structure greatly simplifies the optical path design, and the system size and complexity are significantly reduced.

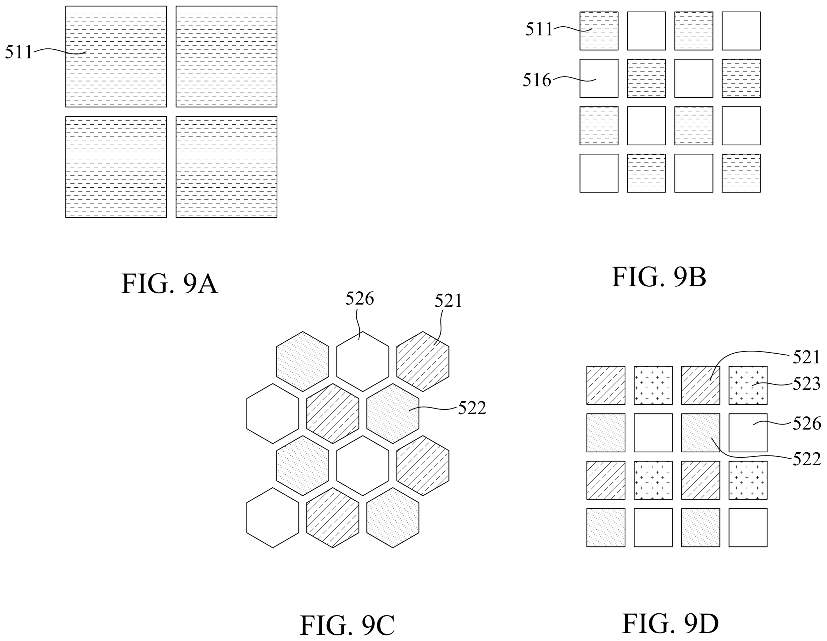

FIG. 9A is a top view exemplarily showing a portion 9A of the micro-LED array 640.In some embodiments, the micro-LED array 640 includes an array constituted by a plurality of light emitting units 511 of single-color micro-LEDs.

FIG. 9B is a top view exemplarily showing the portion 9A of the micro-LED array 640.In some embodiments, the micro-LED array 640 includes an integrated array constituted by a plurality of light emitting units 511 of single-color micro-LEDs and a plurality of image sensors 516.

FIG. 9C is a top view exemplarily showing a portion 9C of the OLED array 650.In some embodiments, the OLED array 650 includes an integrated array constituted by a plurality of light emitting units 521 of single-color OLEDs, a plurality of light emitting units 522 of single-color OLEDs, and a plurality of image sensors 526.

FIG. 9D is a top view exemplarily showing the portion 9C of the OLED array 650.In some embodiments, the OLED array 650 includes an integrated array constituted by a plurality of light emitting units 521 of single-color OLEDs, a plurality of light emitting units 522 of single-color OLEDs, a plurality of light emitting units 523 of single-color OLEDs, and a plurality of image sensors 526.

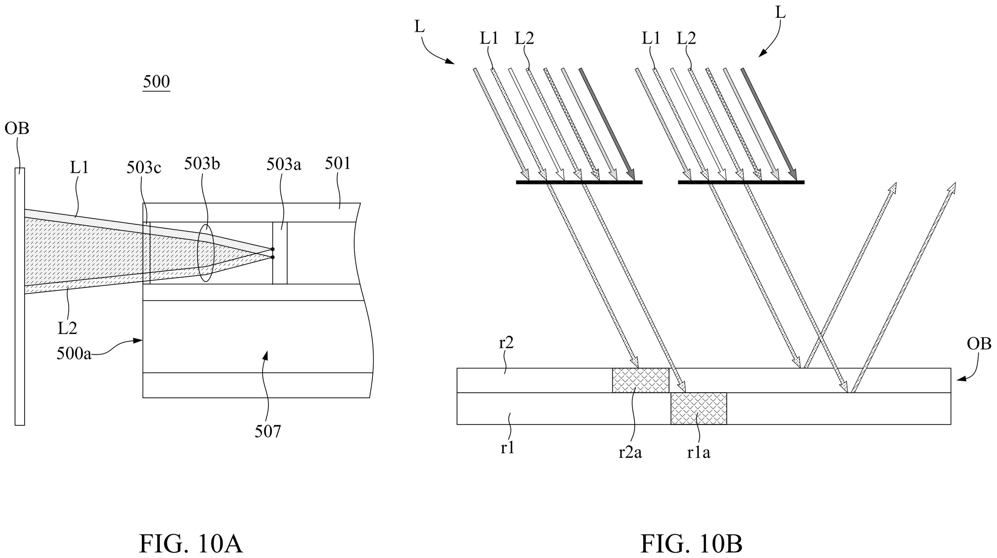

FIG. 10A is a diagram exemplarily showing a light path of the distal system 500.FIG. 10B is a diagram exemplarily showing a light path of the distal system 500.In some embodiments, FIG. 10A illustrates the light path in which light L having multiple specific bands emitted from a multicolor light source (which, for example, has light L1 and light L2 with different wavelengths) is irradiated onto the object under test OB, and FIG. 10B illustrates the light paths that enable the tissue r1a and r2a in regions r1 and r2 at different depths of the object to be sensed. In some embodiments, with the light L having multiple specific bands emitted from the multicolor light source, the tissue r1a and r2a in the regions r1 and r2 at different depths of the object OB can be sensed, and then the processor and software receive and process the image captured by the camera to obtain the image information. Different layers of biological tissue (i.e., different depths, such as the regions r1 and r2) have different transmittances and absorptivities for different specific wavelengths of light. By emitting light L having multiple specific bands (for example, the light L1 and the light L2), it is possible to selectively penetrate to different depths of the object OB. When light of a specific wavelength is absorbed and does not penetrate, for example, strongly absorbed by hemoglobin, it indicates that blood vessels may be present in that region. Dense blood vessels are usually a feature of tumors, so, by observing the absorption of light, it is possible to determine whether a tumor is present. For example, blue-violet light in the wavelength range from 400 nm to 430 nm has the shallowest penetration depth and is strongly absorbed by hemoglobin, and is suitable for displaying details of superficial microvessels and mucosas for detecting early cancers or epithelial lesions. On the other hand, red light in the wavelength range of from 620 nm to 700 nm has the deepest penetration, and is used for observing submucosal structures and deep vessels.

In some embodiments, the wavelength ranges, colors, penetration depths, and main applications are summarized in the table below.

| Wavelength Range (nm) | Color | Penetration Depth | Main Application |
|---|---|---|---|
| 400-430 | blue-violet light | shallowest layer | strong heme absorption; highlights superficial microvessels and mucosal details; early cancer/epithelial lesion detection |
| 450-470 | blue light | shallow layer | relatively strong heme absorption; highlights superficial vessels and structural details; algorithm-enhanced sharpness; adjustable white balance |
| 500-550 | green light | middle layer | moderate heme absorption; visualizes mucosa and submucosal vessels; often used with blue light for high contrast and white balance adjustment |
| 580-600 | amber light | intermediate-deep layer | weak heme absorption but good contrast; suitable for submucosal tumor visualization, deep vessel patterns, tumor invasion depth |
| 620-700 | red light | deep layer | deepest penetration; visualizes submucosal structures, large vessels, bleeding source localization; used in near-infrared endoscopy |

In some embodiments, the imaging system needs two main light sources, one is for structured light to observe the surface 3D appearance, and the other one is for NBI light to observe deep lesions. The two received images may be combined on the AR glasses to achieve "naked-eye 3D navigation".

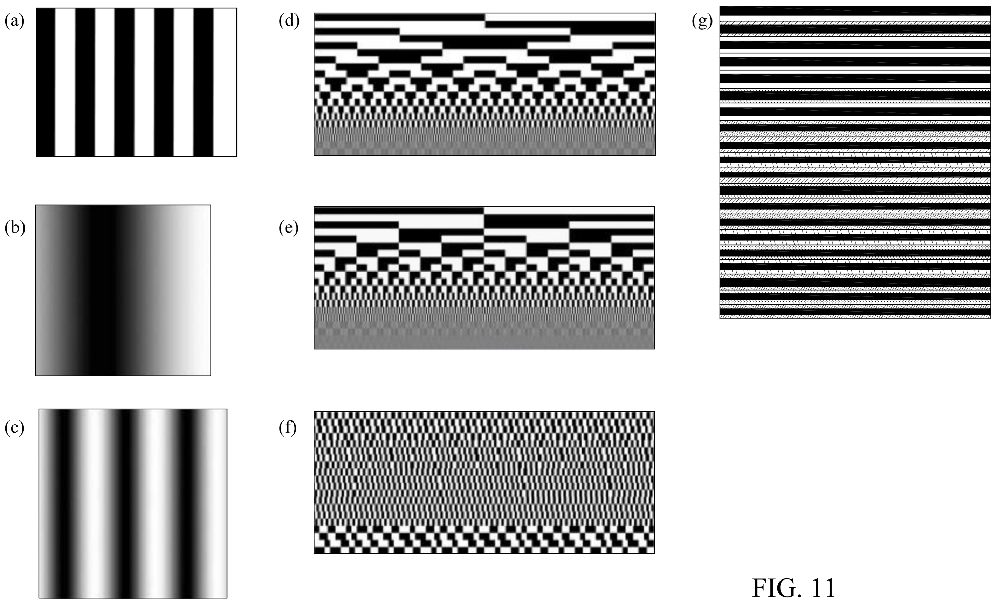

FIG. 11, (a) to (g), are diagrams exemplarily showing patterns of structured light. In some embodiments, the light emitting device having the multicolor light source may further include a structured light source. In some embodiments, the structured light source may be provided by a micro-LED array, an OLED array, or a combination of both. In some embodiments, the structured light may have a light emitting wavelength in a range from ultraviolet to near-infrared light. In some embodiments, the structured light (for example, the patterns such as stripes and gratings as shown in FIG. 11, (a) to (g)) is specifically used to identify the 3D structure of lesions, such as early cancer (its 3D size is less than 1 cm), capillaries (its 3D size is 5 µm to 10 µm), small veins/arterioles (its 3D size is 10 µm to 100 µm), and intestinal and lower esophageal microvessels (its 3D size is 20 µm to 50 µm). In some embodiments, the pattern of the structured light source may have a stripe spacing from 5 µm to 20 µm and a projection distance from 10 µm to 15 mm. In some embodiments, the spacing of the structured light projected onto a target object, magnified by a lens, is 50 µm to 200 µm, and the total number of projected stripes are about 20 to 30. In some embodiments, single-color light is used to determine whether a symptom is present, while the structured light is used to identify the 3D structure of a symptom.

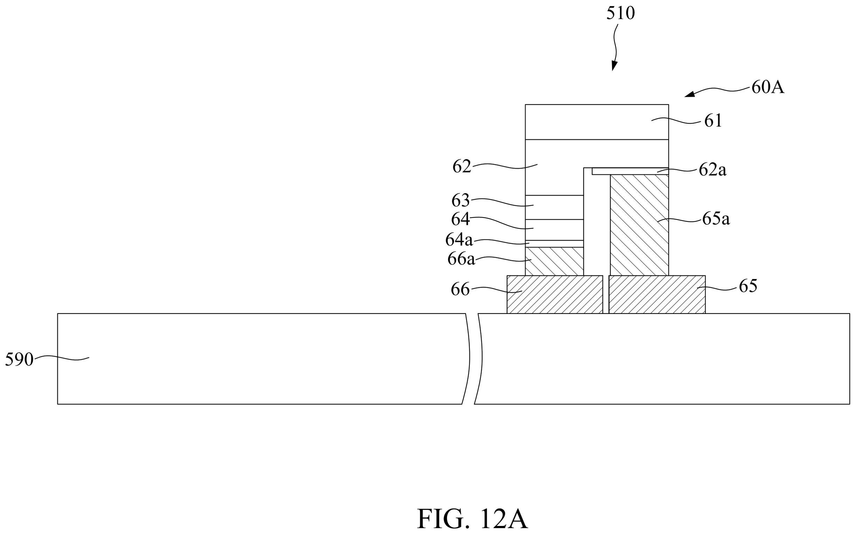

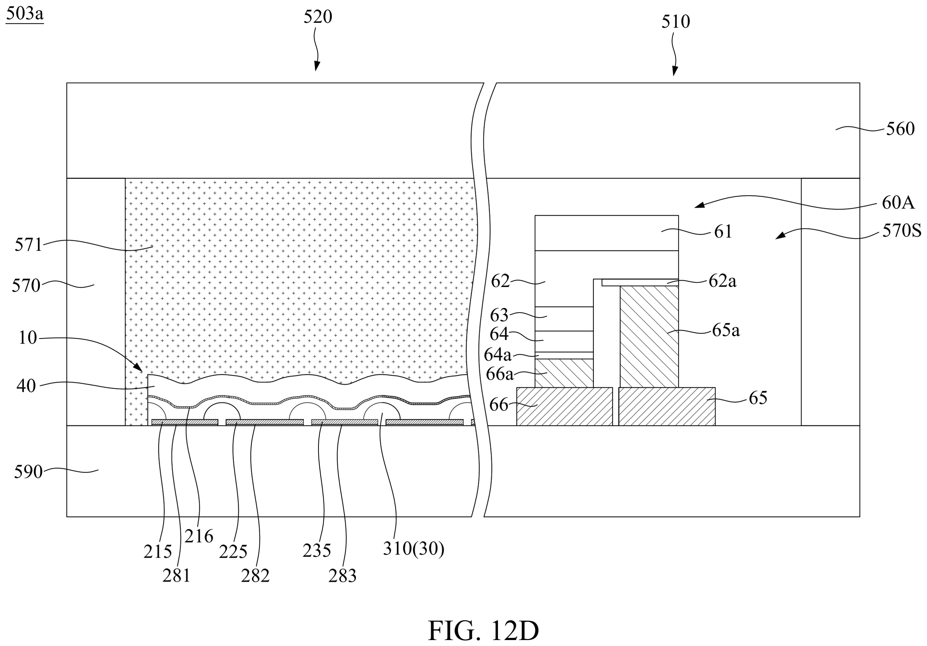

FIGS. 12A-12D are diagrams exemplarily showing a method for manufacturing the imaging system.

Referring to FIG. 5A and FIG. 5B, the method for manufacturing the imaging system may include manufacturing a distal system 500, and the step of manufacturing the distal system 500 may include providing a housing 501 having hollow conduits 503 and 504 and working channels 507 and 508.

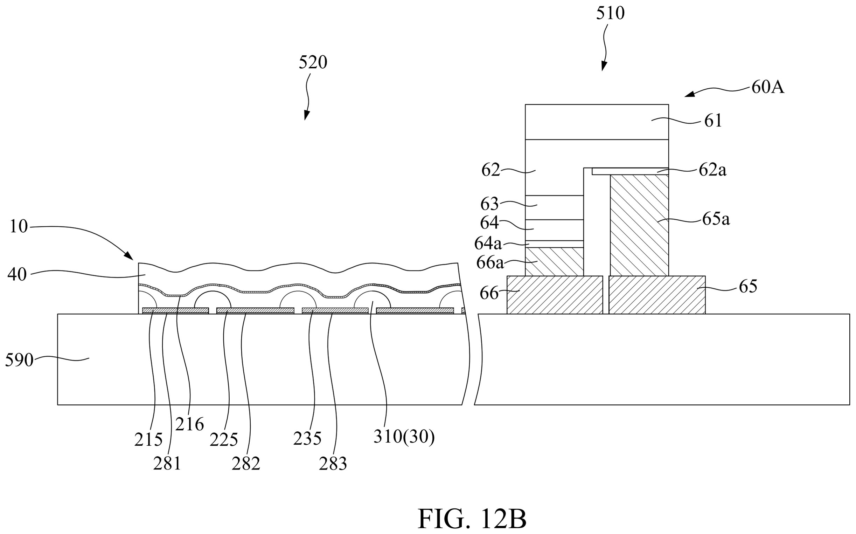

Referring to FIG. 12A and FIG. 12B, the method for manufacturing the imaging system may include manufacturing a light emitting device 503a, which includes an OLED, a micro-LED, a QLED, or any combination thereof. In some embodiments, manufacturing the light emitting device 503a may include following steps: providing a substrate 590; disposing an OLED array 520 including an OLED 10 and a micro-LED array 510 including a micro-LED 60A on the substrate 590; and covering a transparent encapsulation layer 571 on the OLED array 520 and exposing the micro-LED array 510. In some embodiments, manufacturing the light emitting device 503a may include following steps as will be described with reference to FIGS. 12A-12D.

Referring to FIG. 12A, the micro-LED array 510 including the micro-LED 60A may be disposed on the substrate 590.

Referring to FIG. 12B, the OLED array 520 including the OLED 10 may be disposed on the substrate 590.

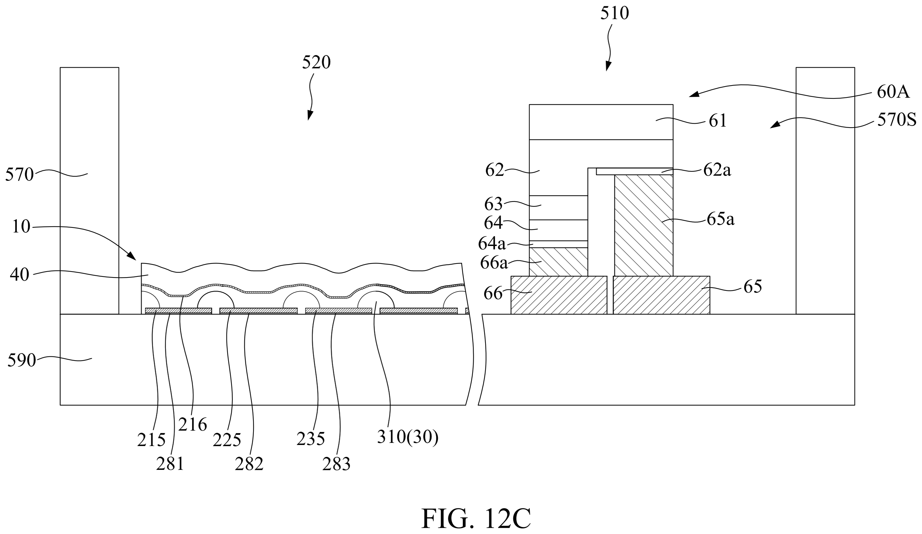

Referring to FIG. 12C, a dam structure 570 may be manufactured. In some embodiments, the dam structure 570 surrounds the micro-LED array 510 and the OLED array 520.

In some embodiments, the transparent encapsulation layer 571 may cover the OLED array 520 and expose the micro-LED array 510. In some embodiments, a transparent cover plate 560 is attached to the dam structure 570 and covers the micro-LED array 510 and the OLED array 520. In some embodiments, the micro-LED 60A is exposed in space 570S defined by the dam structure 570 and the transparent cover plate 560. In some embodiments, the transparent encapsulation layer 571 has a certain viscosity, which can achieve local coverage through inherent viscosity, for example, covering only the OLED array 520 and exposes the micro-LED array 510.

In some embodiments, referring to FIG. 5A and FIG. 5B, the light emitting device 503a may be disposed in the hollow conduit 503, and the distal system 500 may be electrically connected to a processor. In some embodiments, a lens 503b is disposed between an opening of the hollow conduit 503 and the light emitting device 503a, and the opening of the hollow conduit 503 is covered with a transparent protection plate 503c. In some embodiments, an image sensor structure 504a is disposed within the hollow conduit 504 of the housing 501 of the distal system 500.

In some embodiments, referring to FIG. 6B, a microlens structure 550 may be disposed on a light emitting surface of the OLED 10, and a microlens structure 550a may be disposed on a light emitting surface of the micro-LED 60A.

In some embodiments, referring to FIG. 8A and FIG. 8B, a distal system 600 may be manufactured, a light emitting device 603a may be disposed in the hollow conduit 603, and the distal system 600 may be electrically connected to a processor. In some embodiments, a lens 603b may be disposed between an opening of the hollow conduit 603 and the light emitting device 603a, and the opening of the hollow conduit 603 is covered with a transparent protection plate 603c. In some embodiments, a plurality of image sensors are integrated into the OLED array 650, and a plurality of image sensors are integrated into the micro-LED array 640, so as to manufacture the light emitting device 603a.

The aforementioned content generally outlines the features of some implementations, allowing one skilled in the art to better understand various aspects of the disclosure. One skilled in the art should understand that hid disclosure can be easily used as a foundation to design or modify other processes and structures to achieve the same objectives and/or attain the same advantages as the embodiments described in the present application. One skilled in the art should also understand that such equivalent structures do not depart from the spirit and the scope of the disclosed content, and various changes, substitutions, and modifications can be made without departing from the spirit and the scope of the disclosure.

## Claims

1. An imaging system, comprising:
a processor (1001b3); and
a distal system (500, 600, 1001a1, 1002a, 1002a', 1002c) electrically connected to the processor, the distal system comprising an image sensor structure (504a) and a light emitting device (503a, 503a'), wherein the light emitting device comprises an organic light emitting device, a micro light emitting diode, a quantum dot light emitting diode, or any combination thereof, wherein the organic light emitting device (10, 10A, 10B) comprises:
an electrode (215);
a semi-reflective surface (281a/290a) and a fully reflective surface (2162/2151); and
an organic light emitting layer (260A) disposed above the electrode and between the semi-reflective surface and the fully reflective surface, wherein a distance between the semi-reflective surface and the fully reflective surface defines a micro resonant cavity.

2. The imaging system according to claim 1, wherein the light emitting device further comprises a first microlens structure (550) disposed on a light emitting surface of the organic light emitting device and a second microlens structure (550a) disposed on a light emitting surface of the micro light emitting diode.

3. The imaging system according to claim 1, wherein the light emitting device is configured to switchably emit colored light having two or more colors, NBI light having a full width at half maximum less than 30 nm, and structured light having a predetermined pattern.

4. The imaging system according to claim 1, wherein the light emitting device further comprises a substrate (590), an organic light emitting device array (520) disposed on the substrate, and a micro light emitting diode array (510) disposed on the substrate, the organic light emitting device array comprises the organic light emitting device, and the micro light emitting diode array comprises the micro light emitting diode.

5. The imaging system according to claim 4, wherein the micro light emitting diode array (510) comprises a plurality of the micro light emitting diodes, and light emitted from the micro light emitting diode array having one or more colors is used to compensate for luminous efficiency of light emitted from a plurality of the organic light emitting devices of the organic light emitting device array (520) having one or more colors.

6. The imaging system according to claim 4, wherein the light emitting device further comprises a transparent encapsulation layer (571) covering the organic light emitting device array (520) and exposing the micro light emitting diode array (510).

7. The imaging system according to claim 4, wherein the image sensor structure (504a) comprises a plurality of first image sensors integrated into the organic light emitting device array (520) and a plurality of second image sensors integrated into the micro light emitting diode array (510), and the first image sensors and the second image sensors are all disposed on the substrate.

8. The imaging system according to claim 1, wherein the distal system further comprises a first hollow conduit (503) and a second hollow conduit (504), the light emitting device (503a) is disposed in the first hollow conduit (503), and the image sensor structure (504a) is disposed in the second hollow conduit (504).

9. The imaging system according to claim 8, wherein the distal system further comprises a transparent protection plate (503c) covering an opening of the first hollow conduit and a lens (503b) disposed between the transparent protection plate and the light emitting device.

10. The imaging system according to claim 1, wherein the distal system is configured to capture image information of an object under test and transmit the image information to the processor (1001b3), and the processor is configured to process the image information to generate an image of an appearance of the object or a 3D structural simulation result of the object.

11. The imaging system according to claim 10, further comprising an augmented reality glasses (1001b1, 1002b, 1002d), wherein the augmented reality glasses comprises the processor.

12. The imaging system according to claim 10, further comprising an augmented reality glasses (1002b, 1002d), wherein the augmented reality glasses is configured to receive the image of the appearance of the object and/or the 3D structural simulation result of the object, and after processed by the processor using a swarm algorithm, to present a real-time image in a display area of the augmented reality glasses.

13. The imaging system according to claim 1, wherein the distal system (1001a1) comprises a colored light source (1001a2), an NBI light source (1001a3), and a structured light source (1001a4), and the image sensor structure is configured to capture image information.

14. The imaging system according to claim 1, wherein the processor is configured to transmit a control signal to control the organic light emitting device to switch between emitting colored light (1002a1), NBI light (1002a2), and structured light (1002c1), so as to implement a colored light source, a NBI light source, and a structured light source of the distal system.

15. The imaging system according to claim 1, wherein the distal system (500, 600, 1001a1, 1002a, 1002a', 1002c) does not comprise an optical fiber, a light emitting diode, or a color filter.
